# EUROPEAN PATENT SPECIFICATION

(11) **EP 1 856 298 B1**
(45) Date of publication and mention of the grant of the patent: **27.09.2017**
(21) Application number: 06735264.1
(22) Date of filing: 16.02.2006
(51) Int. Cl.: C12Q 1/70

(54) **METHODS AND COMPOSITIONS FOR DETERMINING ANTI-HIV DRUG SUSCEPTIBILITY AND REPLICATION CAPACITY OF HIV**
VERFAHREN UND ZUSAMMENSETZUNGEN ZUR BESTIMMUNG DER SUSZEPTIBILITÄT FÜR ANTI-HIV-MITTEL UND DES HIV-REPLIKATIONSVERMÖGENS
PROCÉDÉS ET COMPOSITIONS POUR LA DÉTERMINATION DE LA SENSIBILITÉ ET DE LA CAPACITÉ DE RÉPLICATION DE HIV DE MÉDICAMENT ANTI-VIH

(30) Priority: 18.02.2005 US 654238 P
(43) Date of publication of application: 21.11.2007
(60) Divisional application: 12167177.0
(73) Proprietor: Monogram BioSciences, Inc., South San Francisco, CA 94080-4811 (US)
(72) Inventor: PAXINOS, Ellen, San Jose, California 95117 (US); FRANSEN, Signe, San Francisco, California 94131 (US); PETROPOULOS, Christos, J., Half Moon Bay, California 94106 (US)
(74) Representative: Donald, Jenny Susan
(86) International application number: PCT/US2006/005512
(87) International publication number: WO 2006/089046

(56) References cited:
- WO-A2-2006/133267
- US-A- 5 837 464
- JARMY GERGELY ET AL: "Phenotypic analysis of the sensitivity of HIV-1 to inhibitors of the reverse transcriptase, protease, and integrase using a self-inactivating virus vector system" JOURNAL OF MEDICAL VIROLOGY, vol. 64, no. 3, July 2001 (2001-07), pages 223-231, XP002564936 ISSN: 0146-6615
- Fransen et al.: "INTEGRASE INHIBITOR SUSCEPTIBILITY CAN BE MEASURED USING RECOMBINANT VIRUSES THAT EXPRESS PATIENT VIRUS INTEGRASE ALONE, OR IN COMBINATION WITH PROTEASE AND REVERSE TRANSCRIPTASE" 22 February 2005 (2005-02-22), XP002564937 Retrieved from the Internet: URL:http://www.aegis.com/conferences/croi/ 2005/Abstract_725.pdf> [retrieved on 2010-01-22]
- DATABASE EMBL [Online] 10 March 1994 (1994-03-10), "HIV-1 proviral vif gene DNA" XP002564938 retrieved from EBI accession no. EMBL:Z30642 Database accession no. Z30642
- DATABASE Geneseq [Online] 29 January 1999 (1999-01-29), XP002564939 retrieved from EBI accession no. GSN:AAV64757 Database accession no. AAV64757
- DATABASE EMBL [Online] 3 February 2000 (2000-02-03), "HIV-1 FIN93210 from Finland gag protein (gag) gene, partial cds." XP002564940 retrieved from EBI accession no. EMBL:AF219286 Database accession no. AF219286
- PETROPOULOS ET AL.: 'A Novel Phenotypic Drug Susceptibility Assay for Human Immunodeficiency Virus Type 1.' ANTIMICROBIAL AGENTS AND CHEMOTHERAPY vol. 44, no. 4, April 2000, pages 920 - 928, XP002962272
- QARI ET AL.: 'Comparative Analysis of Two Commercial Phenotypic Assays for Drug Susceptibility Testing of Human Immunodeficiency Virus Type 1.' JOURNAL OF CLINICAL MICROBIOLOGY vol. 40, no. 1, 2002, pages 31 - 35, XP008122174
- HAZUDA D J ET AL: "Inhibitors of strand transfer that prevent integration and inhibit HIV - 1 replication in cells", SCIENCE, AMERICAN ASSOCIATION FOR THE ADVANCEMENT OF SCIENCE, WASHINGTON, DC; US, vol. 287, 28 January 2000 (2000-01-28), pages 646-650, XP002175834, ISSN: 0036-8075, DOI: 10.1126/SCIENCE.287.5453.646
- H. Chen ET AL: "Characterization of a Replication-Defective Human Immunodeficiency Virus Type 1 att Site Mutant That Is Blocked after the 3' Processing Step of Retroviral Integration", Journal of Virology, vol. 74, no. 17, 1 September 2000 (2000-09-01), pages 8188-8193, XP055112864, ISSN: 0022-538X, DOI: 10.1128/JVI.74.17.8188-8193.2000
- H. Chen ET AL: "Multiple Integrase Functions Are Required to Form the Native Structure of the Human Immunodeficiency Virus Type I Intasome", Journal of Biological Chemistry, vol. 274, no. 24, 11 June 1999 (1999-06-11), pages 17358-17364, XP055112866, ISSN: 0021-9258, DOI: 10.1074/jbc.274.24.17358
- Min Li ET AL: "Retroviral DNA integration: reaction pathway and critical intermediates", The EMBO Journal, vol. 25, no. 6, 22 March 2006 (2006-03-22) , pages 1295-1304, XP055112871, ISSN: 0261-4189, DOI: 10.1038/sj.emboj.7601005
- WEI S-Q ET AL: "A large nucleoprotein assembly at the ends of the viral DNA mediates retroviral DNA integration", EMBO JOURNAL, OXFORD UNIVERSITY PRESS, SURREY, GB, vol. 16, no. 24, 15 December 1997 (1997-12-15), pages 7511-7520, XP002331097, ISSN: 0261-4189, DOI: 10.1093/EMBOJ/16.24.7511
- BERA S ET AL: "Molecular Interactions between HIV-1 Integrase and the Two Viral DNA Ends within the Synaptic Complex that Mediates Concerted Integration", JOURNAL OF MOLECULAR BIOLOGY, ACADEMIC PRESS, UNITED KINGDOM, vol. 389, no. 1, 29 May 2009 (2009-05-29), pages 183-198, XP026469147, ISSN: 0022-2836, DOI: 10.1016/J.JMB.2009.04.007 [retrieved on 2009-04-09]
- E. A. Hehl ET AL: "Interaction between Human Immunodeficiency Virus Type 1 Reverse Transcriptase and Integrase Proteins", Journal of Virology, vol. 78, no. 10, 15 May 2004 (2004-05-15), pages 5056-5067, XP055112874, ISSN: 0022-538X, DOI: 10.1128/JVI.78.10.5056-5067.2004
- Alon Herschhorn ET AL: "Quantitative analysis of the interactions between HIV-1 integrase and retroviral reverse transcriptases", Biochemical Journal, vol. 412, no. 1, 15 May 2008 (2008-05-15), pages 163-170, XP055112877, ISSN: 0264-6021, DOI: 10.1042/BJ20071279
- Iris Oz ET AL: "Inhibition of the integrases of human immunodeficiency viruses type 1 and type 2 by reverse transcriptases", Biochemical Journal, vol. 361, no. 3, 1 February 2002 (2002-02-01), pages 557-566, XP055112879, ISSN: 0264-6021, DOI: 10.1042/0264-6021:3610557
- TASARA<A> T ET AL: "HIV-1 reverse transcriptase and integrase enzymes physically interact and inhibit each other", FEBS LETTERS, ELSEVIER, AMSTERDAM, NL, vol. 507, no. 1, 19 October 2001 (2001-10-19), pages 39-44, XP004311108, ISSN: 0014-5793, DOI: 10.1016/S0014-5793(01)02945-3
- T. A. Wilkinson ET AL: "Identifying and Characterizing a Functional HIV-1 Reverse Transcriptase-binding Site on Integrase", Journal of Biological Chemistry, vol. 284, no. 12, 20 March 2009 (2009-03-20), pages 7931-7939, XP055112882, ISSN: 0021-9258, DOI: 10.1074/jbc.M806241200
- J Virol ET AL: "Transcription Complex with the Nucleoprotein Reverse Transcription through Specific Interactions Integrase Protein Promotes Reverse Human Immunodeficiency Virus Type 1", , 1 January 1999 (1999-01-01), page 2126, XP055112884, Retrieved from the Internet: URL:http://jvi.asm.org/content/73/3/2126.f ull.pdf [retrieved on 2014-04-08]
- K. Zhu ET AL: "Requirement for Integrase during Reverse Transcription of Human Immunodeficiency Virus Type 1 and the Effect of Cysteine Mutations of Integrase on Its Interactions with Reverse Transcriptase", Journal of Virology, vol. 78, no. 10, 27 April 2004 (2004-04-27), pages 5045-5055, XP055112886, ISSN: 0022-538X, DOI: 10.1128/JVI.78.10.5045-5055.2004
- J Virol ET AL: "by the Viral Nucleocapsid Protein by Purified Integrase In Vitro: Stimulation Ends Immunodeficiency Virus Type 1 cDNA Coupled Integration of Human", , 1 January 1999 (1999-01-01), page 6670, XP055112890, Retrieved from the Internet: URL:http://jvi.asm.org/content/73/8/6670.f ull.pdf [retrieved on 2014-04-08]
- POLJAK L ET AL: "Analysis of NCp7-dependent Activation of HIV-1 cDNA Integration and its Conservation Among Retroviral Nucleocapsid Proteins", JOURNAL OF MOLECULAR BIOLOGY, ACADEMIC PRESS, UNITED KINGDOM, vol. 329, no. 3, 6 June 2003 (2003-06-06), pages 411-421, XP004457674, ISSN: 0022-2836, DOI: 10.1016/S0022-2836(03)00472-8
- M. S. Briones ET AL: "Role of Human Immunodeficiency Virus Type 1 Integrase in Uncoating of the Viral Core", Journal of Virology, vol. 84, no. 10, 10 March 2010 (2010-03-10), pages 5181-5190, XP055112891, ISSN: 0022-538X, DOI: 10.1128/JVI.02382-09

## Description

### 1. FIELD OF INVENTION

This invention relates, in part, to methods for determining the susceptibility of a human immunodeficiency virus ("HIV") to an integrase inhibitor.

### 2. BACKGROUND OF THE INVENTION

More than 60 million people have been infected with the human immunodeficiency virus ("HIV"), the causative agent of acquired immune deficiency syndrome ("AIDS"), since the early 1980s. *See* Lucas, 2002, Lepr Rev. 73(1):64-71. HIV/AIDS is now the leading cause of death in sub-Saharan Africa, and is the fourth biggest killer worldwide. At the end of 2001, an estimated 40 million people were living with HIV globally. *See* Norris, 2002, Radiol Technol. 73(4):339-363.

Modern anti-HIV drugs target different stages of the HIV life cycle and a variety of enzymes essential for HIV's replication and/or survival. Amongst the drugs that have so far been approved for AIDS therapy are nucleoside reverse transcriptase inhibitors ("NRTIs") such as AZT, ddI, ddC, d4T, 3TC, and abacavir; nucleotide reverse transcriptase inhibitors such as tenofovir; non-nucleoside reverse transcriptase inhibitors ("NNRTIs") such as nevirapine, efavirenz, and delavirdine; protease inhibitors ("PIs") such as saquinavir, ritonavir, indinavir, nelfinavir, amprenavir, lopinavir and atazanavir; and fusion inhibitors, such as enfuvirtide. In addition, experiments are currently underway to identify anti-HIV drugs that target other HIV polypeptide activities, including, for example, the activities of integrase and RNAse H. For example, the naphthyridine carboxamide L-870,810 (Merck, Inc., Whitehouse Station, NJ) is currently being investigated as a potential integrase inhibitor. *See* Hazuda et al., 2004, P.N.A.S. USA 101:11233-11238.

Nonetheless, in the vast majority of subjects none of the antiviral drugs currently approved, either alone or in combination, proves effective either to prevent eventual progression of chronic HIV infection to AIDS or to treat acute AIDS. This phenomenon is due, in part, to the high mutation rate of HIV and the rapid emergence of mutant HIV that are resistant to antiviral therapeutics upon administration of such drugs to infected individuals. Accordingly, as new drugs targeting new HIV polypeptides become available, phenotypic assays for determining resistance or susceptibility of HIV infecting a patient to such new anti-HIV drugs are needed. This and other needs are provided by the present invention.

### 3. SUMMARY OF THE INVENTION

In certain aspects, the present invention provides a method for determining the susceptibility of a human immunodeficiency virus (HIV) to an integrase inhibitor in accordance with the claims. In certain embodiments, the method comprises culturing a host cell comprising a patient-derived segment and an indicator gene in the presence of the integrase inhibitor, measuring the activity of the indicator gene in the host cell; and comparing the activity of the indicator gene as measured with a reference activity of the indicator gene, wherein the difference between the measured activity of the indicator gene relative to the reference activity correlates with the susceptibility of the HIV to the integrase inhibitor, thereby determining the susceptibility of the HIV to the integrase inhibitor. In certain embodiments, the activity of the indicator gene depends on the activity of the HIV integrase encoded by the patient-derived segment. In certain embodiments, the patient-derived segment comprises a nucleic acid sequence that encodes HIV reverse transcriptase, integrase and RNAse H.

In certain embodiments, the method additionally comprises the step of infecting the host cell with a viral particle comprising the patient-derived segment and the indicator gene prior to culturing the host cell. In certain embodiments, the indicator gene is a luciferase gene.

In another aspect, the disclosure provides a vector comprising a patient-derived segment and an indicator gene. In certain embodiments, the patient-derived segment comprises a nucleic acid sequence that encodes HIV integrase or RNAse H. In certain embodiments, the activity of the indicator gene depends on the activity of the HIV integrase or RNAse H.

In another aspect, the invention provides a method for determining resistance of an HIV infecting a patient to an integrase inhibitor. In certain embodiments, the method comprises determining the susceptibility of the HIV to the integrase inhibitor according to a method of the invention, and comparing the determined susceptibility of the HIV to the integrase inhibitor with a standard curve of susceptibility of the HIV to the integrase inhibitor. In certain embodiments, a decrease in the susceptibility of the HIV to the integrase inhibitor relative to the standard curve indicates that the HIV is resistant to the integrase inhibitor . In certain embodiments, the amount of the decrease in susceptibility of the HIV to the integrase inhibitor indicates the degree to which the HIV is resistant to the integrase inhibitor.

In another aspect, the invention provides a method for determining the progression or development of resistance of an HIV infecting a patient to an integrase inhibitor. In certain embodiments, the method comprises determining the susceptibility of the HIV to the integrase inhibitor at a first time according to a method of the invention; assessing the effectiveness of the integrase inhibitor according to a method of the invention at a later second time; and comparing the effectiveness of the integrase inhibitor assessed at the first and second time. In certain embodiments, a patient-derived segment is obtained from the patient at about the first time. In certain embodiments, a decrease in the susceptibility of the HIV to the integrase inhibitor at the later second time as compared to the-first time indicates development or progression of integrase inhibitor resistance in the HIV infecting the patient.

In another aspect, the disclosure provides a method for determining the replication capacity of a human immunodeficiency virus (HIV). In certain embodiments, the method comprises culturing a host cell comprising a patient-derived segment and an indicator gene, measuring the activity of the indicator gene in the host cell, wherein the activity of the indicator gene between the activity of the indicator gene measured in step (b) relative to a reference activity indicates the replication capacity of the HIV, thereby determining the replication capacity of the HIV. In certain embodiments, the activity of the indicator gene depends on the activity of a polypeptide encoded by the patient-derived segment. In certain embodiments, the patient-derived segment comprises a nucleic acid sequence that encodes integrase or RNAse H.

In another aspect, the present disclosure provides a method for determining the effectiveness of a candidate anti-HIV compound. In certain embodiments, the method comprises culturing a host cell comprising a patient-derived segment and an indicator gene in the presence of the candidate anti-HIV compound, measuring the activity of the indicator gene in the host cell; and comparing the activity of the indicator gene as measured with a reference activity of the indicator gene, wherein the difference between the measured activity of the indicator gene relative to the reference activity correlates with the effectiveness of the candidate anti-HIV compound, thereby determining the effectiveness of the candidate anti-compound. In certain embodiments, the activity of the indicator gene depends on the activity of a polypeptide encoded by the patient-derived segment. In certain embodiments, the patient-derived segment comprises a nucleic acid sequence that encodes integrase or RNAse H. In certain embodiments, the reference activity of the indicator gene is determined by performing a method of the disclosure in the absence of the candidate anti-HIV compound.

In another aspect, the invention provides an oligonucleotide that can be used in the methods of the invention. In certain embodiments, the oligonucleotide comprises a nucleic acid sequence selected from the group consisting of SEQ ID NO:1, SEQ ID NO 2:, SEQ ID NO:3, and SEQ ID NO:4.

In another aspect, the invention provides a nucleic acid segment that has been reverse transcribed or amplified with an oligonucleotide of the invention.

In another aspect, the disclosure provides a computer-implemented method for determining the susceptibility of an HIV to an anti-HIV drug. In certain embodiments, the method comprises inputting information regarding the activity of an indicator gene determined according to a method of the disclosure and a reference activity of an indicator gene and instructions to compare the activity of the indicator gene determined according to a method of the disclosure with the reference activity of the indicator gene into a computer memory; and comparing the activity of the indicator gene determined according to a method of the disclosure with the reference activity of the indicator gene in the computer memory, wherein the difference between the measured activity of the indicator gene relative to the reference activity correlates with the susceptibility of the HIV to the anti-HIV drug, thereby determining the susceptibility of the HIV to the anti-HIV drug.

In another aspect, the disclosure provides a computer-implemented method for determining the replication capacity of an HIV. In certain embodiments, the method comprises inputting information regarding the activity of an indicator gene determined according to a method of the disclosure and a reference activity of an indicator gene and instructions to compare the activity of the indicator gene determined according to a method of the disclosure with the reference activity of the indicator gene into a computer memory; and comparing the activity of the indicator gene determined according to a method of the disclosure with the reference activity of the indicator gene in the computer memory, wherein the comparison of the measured activity of the indicator gene relative to the reference activity indicates the replication capacity of the HIV, thereby determining the replication capacity of the HIV.

### 4. BRIEF DESCRIPTION OF THE FIGURES

Figure 1a presents a diagrammatic representation of two alternate DNA constructs comprising patient-derived segments.
Figure 1b presents a diagrammatic representation of an assay for assessing anti-HIV drug susceptibility or HIV replication capacity.
Figure 2 presents graphical representations of reduced susceptibility of HIV mutants comprising site-directed mutations in integrase relative to HIV strain IIIB.
Figure 3 presents a tabular comparison of susceptibility of HIV mutants comprising site-directed mutations determined according to the assays described herein with susceptibilities of such HIV mutants determined by Hazuda et al., 2004, P.N.A.S. USA 101:11233-11238.
Figure 4 presents a graphical representation of the distribution of the fold changes (FC) in IC₅₀ relative to HIV strain IIIb of 45 HIV isolated from treatment-naïve patients.
Figure 5 presents a comparison of the fold change observed in IC₅₀ for 30 clinical isolates from treatment-naive patients for two different patient-derived segments, POL and RHIN; the mean FC observed was 0.86 for the RHIN segment and 0.85 for the POL segment, the median FC observed was 0.82 for the RHIN segment and 0.85 for the POL segment, and the mean FC observed for the two assays was not significantly different, with t test p value > 0.05.

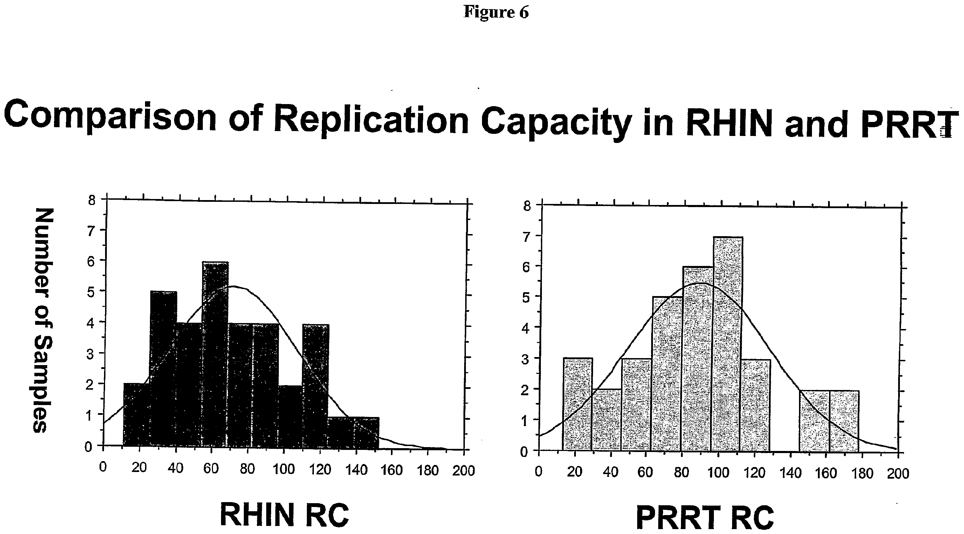

Figure 6 presents a comparison of replication capacities determined for 33 clinical isolates from treatment-naive patients for two different patient-derived segments, RHIN, prepared as described herein and PR-RT, prepared as described in U.S. Patent No. 5,837,464; the mean RC observed was 71 for the RHIN segment and 88.5 for the PR-RT segment; the median RC observed was 63 for the RHIN segment and 94 for the PR-RT segment, the range of observed RC was 11 to 152 for the RHIN segment and 13 to 178 for the PR-RT segment, and mean FC observed for the two assays was not significantly different, with t test p value > 0.05.

### 4. DETAILED DESCRIPTION OF THE INVENTION

The present invention provides, *inter alia,* methods for determining the susceptibility to an integrase inhibitor. The methods, and compositions useful in performing the methods, are described extensively below.

### 4.1. Abbreviations

"IN" is an abbreviation for "integrase."
"RH" is an abbreviation for "RNAse H."
"PR" is an abbreviation for "protease."
"RT" is an abbreviation for "reverse transcriptase."
"PCR" is an abbreviation for "polymerase chain reaction."
"HIV" is an abbreviation for human immunodeficiency virus.

The amino acid notations used herein for the twenty genetically encoded L-amino acids are conventional and are as follows:

| **Amino Acid** | **One-Letter Abbreviation** | **Three Letter Abbreviation** |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

Unless noted otherwise, when polypeptide sequences are presented as a series of one-letter and/or three-letter abbreviations, the sequences are presented in the N -> C direction, in accordance with common practice.

Individual amino acids in a sequence are represented herein as AN, wherein A is the standard one letter symbol for the amino acid in the sequence, and N is the position in the sequence. Mutations are represented herein as A₁NA₂, wherein A₁ is the standard one letter symbol for the amino acid in the reference protein sequence, A₂ is the standard one letter symbol for the amino acid in the mutated protein sequence, and N is the position in the amino acid sequence. For example, a G25M mutation represents a change from glycine to methionine at amino acid position 25. Mutations may also be represented herein as NA₂, wherein N is the position in the amino acid sequence and A₂ is the standard one letter symbol for the amino acid in the mutated protein sequence (*e.g*., 25M, for a change from the wild-type amino acid to methionine at amino acid position 25). Additionally, mutations may also be represented herein as A₁NX, wherein A₁ is the standard one letter symbol for the amino acid in the reference protein sequence, N is the position in the amino acid sequence, and X indicates that the mutated amino acid can be any amino acid (*e.g*., G25X represents a change from glycine to any amino acid at amino acid position 25). This notation is typically used when the amino acid in the mutated protein sequence is not known, if the amino acid in the mutated protein sequence could be any amino acid, except that found in the reference protein sequence, or if the amino acid in the mutated position is observed as a mixture of two or more amino acids at that position. The amino acid positions are numbered based on the full-length sequence of the protein from which the region encompassing the mutation is derived. Representations of nucleotides and point mutations in DNA sequences are analogous.

The abbreviations used throughout the specification to refer to nucleic acids comprising specific nucleobase sequences are the conventional one-letter abbreviations. Thus, when included in a nucleic acid, the naturally occurring encoding nucleobases are abbreviated as follows: adenine (A), guanine (G), cytosine (C), thymine (T) and uracil (U). Unless specified otherwise, single-stranded nucleic acid sequences that are represented as a series of one-letter abbreviations, and the top strand of double-stranded sequences, are presented in the 5' -> 3' direction.

### 5.2. Definitions

As used herein, the following terms shall have the following meanings:

A "phenotypic assay" is a test that measures a phenotype of a particular virus, such as, for example, HIV, or a population of viruses, such as, for example, the population of HIV infecting a subject. The phenotypes that can be measured include, but are not limited to, the resistance or susceptibility of a virus, or of a population of viruses, to a specific anti-viral agent or that measures the replication capacity of a virus.

A "genotypic assay" is an assay that determines a genotype of an organism, a part of an organism, a population of organisms, a gene, a part of a gene, or a population of genes. Typically, a genotypic assay involves determination of the nucleic acid sequence of the relevant gene or genes. Such assays are frequently performed in HIV to establish, for example, whether certain mutations are associated with drug resistance or hypersusceptibility or altered replication capacity are present.

The term "% sequence identity" is used interchangeably herein with the term "% identity" and refers to the level of amino acid sequence identity between two or more peptide sequences or the level of nucleotide sequence identity between two or more nucleotide sequences, when aligned using a sequence alignment program. For example, as used herein, 80% identity means the same thing as 80% sequence identity determined by a defined algorithm, and means that a given sequence is at least 80% identical to another length of another sequence. Exemplary levels of sequence identity include, but are not limited to, 60, 70, 80, 85, 90, 95, 98% or more sequence identity to a given sequence.

The term "% sequence homology" is used interchangeably herein with the term "% homology" and refers to the level of amino acid sequence homology between two or more peptide sequences or the level of nucleotide sequence homology between two or more nucleotide sequences, when aligned using a sequence alignment program. For example, as used herein, 80% homology means the same thing as 80% sequence homology determined by a defined algorithm, and accordingly a homologue of a given sequence has greater than 80% sequence homology over a length of the given sequence. Exemplary levels of sequence homology include, but are not limited to, 60, 70, 80, 85, 90, 95, 98% or more sequence homology to a given sequence.

Exemplary computer programs which can be used to determine identity between two sequences include, but are not limited to, the suite of BLAST programs, e.g., BLASTN, BLASTX, and TBLASTX, BLASTP and TBLASTN, publicly available on the Internet at the NCBI website. *See also* Altschul et al., 1990, J. Mol. Biol. 215:403-10 (with special reference to the published default setting, i.e., parameters w=4, t=17) and Altschul et al., 1997, Nucleic Acids Res., 25:3389-3402. Sequence searches are typically carried out using the BLASTP program when evaluating a given amino acid sequence relative to amino acid sequences in the GenBank Protein Sequences and other public databases. The BLASTX program is preferred for searching nucleic acid sequences that have been translated in all reading frames against amino acid sequences in the GenBank Protein Sequences and other public databases. Both BLASTP and BLASTX are run using default parameters of an open gap penalty of 11.0, and an extended gap penalty of 1.0, and utilize the BLOSUM-62 matrix. *See id.*

A preferred alignment of selected sequences in order to determine "% identity" between two or more sequences, is performed using for example, the CLUSTAL-X program, operated with default parameters, including an open gap penalty of 10.0, an extended gap penalty of 0.1, and a BLOSUM 30 similarity matrix.

"Polar Amino Acid" refers to a hydrophilic amino acid having a side chain that is uncharged at physiological pH, but which has at least one bond in which the pair of electrons shared in common by two atoms is held more closely by one of the atoms. Genetically encoded polar amino acids include Asn (N), Gln (Q) Ser (S) and Thr (T).

"Nonpolar Amino Acid" refers to a hydrophobic amino acid having a side chain that is uncharged at physiological pH and which has bonds in which the pair of electrons shared in common by two atoms is generally held equally by each of the two atoms (*i.e.,* the side chain is not polar). Genetically encoded nonpolar amino acids include Ala (A), Gly (G), Ile (I), Leu (L), Met (M) and Val (V) .

"Hydrophilic Amino Acid" refers to an amino acid exhibiting a hydrophobicity of less than zero according to the normalized consensus hydrophobicity scale of Eisenberg et al., 1984, J. Mol. Biol. 179:125-142. Genetically encoded hydrophilic amino acids include Arg (R), Asn (N), Asp (D), Glu (E), Gln (Q), His (H), Lys (K), Ser (S) and Thr (T).

"Hydrophobic Amino Acid" refers to an amino acid exhibiting a hydrophobicity of greater than zero according to the normalized consensus hydrophobicity scale of Eisenberg et al., 1984, J. Mol. Biol. 179:125-142. Genetically encoded hydrophobic amino acids include Ala (A), Gly (G), Ile (I), Leu (L), Met (M), Phe (F), Pro (P), Trp (W), Tyr (Y) and Val (V).

"Acidic Amino Acid" refers to a hydrophilic amino acid having a side chain pK value of less than 7. Acidic amino acids typically have negatively charged side chains at physiological pH due to loss of a hydrogen ion. Genetically encoded acidic amino acids include Asp (D) and Glu (E).

"Basic Amino Acid" refers to a hydrophilic amino acid having a side chain pK value of greater than 7. Basic amino acids typically have positively charged side chains at physiological pH due to association with a hydrogen ion. Genetically encoded basic amino acids include Arg (R), His (H) and Lys (K).

A "mutation" is a change in an amino acid sequence or in a corresponding nucleic acid sequence relative to a reference nucleic acid or polypeptide. For embodiments of the invention comprising HIV protease or reverse transcriptase, the reference nucleic acid encoding protease or reverse transcriptase is the protease or reverse transcriptase coding sequence, respectively, present in NL4-3 HIV (GenBank Accession No. AF324493). Likewise, the reference protease or reverse transcriptase polypeptide is that encoded by the NL4-3 HIV sequence. Although the amino acid sequence of a peptide can be determined directly by, for example, Edman degradation or mass spectroscopy, more typically, the amino sequence of a peptide is inferred from the nucleotide sequence of a nucleic acid that encodes the peptide. Any method for determining the sequence of a nucleic acid known in the art can be used, for example, Maxam-Gilbert sequencing (Maxam et al., 1980, Methods in Enzymology 65:499), dideoxy sequencing (Sanger et al., 1977, Proc. Natl. Acad. Sci. USA 74:5463) or hybridization-based approaches (*see e.g.,* Sambrook et al., 2001, Molecular Cloning: A Laboratory Manual, Cold Spring Harbor Laboratory, 3rd ed., NY; and Ausubel et al., 1989, Current Protocols in Molecular Biology, Greene Publishing Associates and Wiley Interscience, NY).

A "mutant" is a virus, gene or protein having a sequence that has one or more changes relative to a reference virus, gene or protein.

The terms "peptide," "polypeptide" and "protein" are used interchangeably throughout.

The term "wild-type" refers to a viral genotype that does not comprise a mutation known to be associated with drug resistance.

The terms "polynucleotide," "oligonucleotide" and "nucleic acid" are used interchangeably throughout.

The terms "RNAse H" and "RNAse H region of reverse transcriptase" and "RNAse H domain of reverse transcriptase" and like terms, as used herein, are used interchangably and refer to the RNAse H domain of HIV reverse transcriptase, found approximately in amino acids 440-560 of p66 reverse transcriptase.

The term "resistance test vector," as used herein, refers to one or more nucleic acid comprising a patient-derived segment and an indicator gene. In the case where the resistance test vector comprises more than one nucleic acid the patient-derived segment may be contained in one nucleic acid and the indicator gene in a different nucleic acid. For example, the indicator gene and the patient-derived segment may be in a single vector, may be in separate vectors, or the indicator gene and/or patient-derived segment may be integrated into the genome of a host cell. The DNA or RNA of a resistance test vector may thus be contained in one or more DNA or RNA molecules.

The term "patient-derived segment," as used herein, refers to one or more nucleic acids that comprise an HIV nucleic acid sequence corresponding to a nucleic acid sequence of an HIV infecting a patient, where the nucleic acid sequence encodes an HIV gene, gene product, or functional viral sequence that is the target of an anti-HIV drug. A "patient-derived segment" can be prepared by an appropriate technique known to one of skill in the art, including, for example, molecular cloning or polymerase chain reaction (PCR) amplification from viral DNA or complementary DNA (cDNA) prepared from viral RNA, present in the cells (e.g. peripheral blood mononuclear cells, PBMC), serum or other bodily fluids of infected patients. A "patient-derived segment" is preferably isolated using a technique where the HIV infecting the patient is not passed through culture subsequent to isolation from the patient, or if the virus is cultured, then by a minimum number of passages to reduce or essentially eliminate the selection of mutations in culture.

The term "functional viral sequence," as used herein, refers to any nucleic acid sequence (DNA or RNA) with functional activity such as enhancers, promoters, polyadenylation sites, sites of action of transacting factors, such as tar and RRE, packaging sequences, integration sequences, or splicing sequences.

The term "indicator or indicator gene," as used herein, refers to a nucleic acid encoding a protein, DNA structure, or RNA structure that either directly or through a reaction gives rise to a measurable or noticeable aspect, *e.g.,* a color or light of a measurable wavelength or, in the case of DNA or RNA used as an indicator, a change or generation of a specific DNA or RNA structure. A preferred indicator gene is luciferase.

### 4.2. Methods of Determining Susceptibility to Anti-HIV Drugs

In certain aspects, the present disclosure provides a method for determining the susceptibility of a human immunodeficiency virus (HIV) infecting a patient to an anti-HIV drug. In certain embodiments, the method comprises culturing a host cell comprising a patient-derived segment and an indicator gene in the presence of the anti-HIV drug, measuring the activity of the indicator gene in the host cell; and comparing the activity of the indicator gene as measured with a reference activity of the indicator gene, wherein the difference between the measured activity of the indicator gene relative to the reference activity correlates with the susceptibility of the HIV to the anti-HIV drug, thereby determining the susceptibility of the HIV to the anti-HIV drug. In certain embodiments, the activity of the indicator gene depends on the activity of a polypeptide encoded by the patient-derived segment. In certain embodiments, the patient-derived segment comprises a nucleic acid sequence that encodes integrase or RNAse H. In certain embodiments, the patient-derived segment is obtained from the HIV.

In certain embodiments, the reference activity of the indicator gene is determined by performing determining the activity of the indicator gene in the absence of the anti-HIV drug. In certain embodiments, the reference activity of the indicator gene is determined by determining the susceptibility of a reference HIV to the anti-HIV agent. In certain embodiments, the reference activity is determined by performing a method of the invention with a standard laboratory viral segment. In certain embodiments, the standard laboratory viral segment comprises a nucleic acid sequence from HIV strain NL4-3 (Genbank Accession No. M19921). In certain embodiments, the standard laboratory viral segment comprises a nucleic acid sequence from HIV strain IIIB (Genbank Accession No. U12055).

In embodiments of the invention, the anti-HIV drug inhibits RNAse H. In certain embodiments, the HIV is determined to have reduced susceptibility to the anti-HIV drug. In certain embodiments, the HIV is determined to have increased susceptibility to the anti-HIV drug. In certain embodiments, the patient-derived segment comprises an *pol* gene, or a portion thereof. In certain embodiments, the patient-derived segment is about 1.8 kB in length. In certain embodiments, the patient-derived segment encodes integrase and RNAse H. In certain embodiments, the patient-derived segment is about 3.3 kB in length. In certain embodiments, the patient-derived segment encodes reverse transcriptase, integrase, and RNAse H.

In certain embodiments, the patient-derived segment has been prepared in a reverse transcription or polymerase chain reaction (PCR) reaction. In certain embodiments, the reverse transcription or PCR reaction comprises an oligonucleotide comprising a nucleic acid sequence that is SEQ ID NO:1, SEQ ID NO:2, SEQ ID NO:3, or SEQ ID NO:4.

In certain embodiments, the method additionally comprises the step of infecting the host cell with a viral particle comprising the patient-derived segment and the indicator gene prior to culturing the host cell.

In certain embodiments, the indicator gene is a luciferase gene. In certain embodiments, the indicator gene is a lacZ gene. In certain embodiments, the host cell is a human cell. In certain embodiments, the host cell is a human embryonic kidney cell. In certain embodiments, the host cell is a 293 cell. In certain embodiments, the host cell is a human T cell. In certain embodiments, the host cell is derived from a human T cell leukemia cell line. In certain embodiments, the host cell is a Jurkat cell. In certain embodiments, the host cell is a H9 cell. In certain embodiments, the host cell is a CEM cell.

In another aspect, the disclosure provides a vector comprising a patient-derived segment and an indicator gene. In certain embodiments, the patient-derived segment comprises a nucleic acid sequence that encodes HIV integrase or RNAse H. In certain embodiments, the activity of the indicator gene depends on the activity of the HIV integrase or RNAse H.

In certain embodiments, the patient-derived segment comprises an HIV pol gene, or a portion thereof. In certain embodiments, the indicator gene is a functional indicator gene. In certain embodiments, indicator gene is a non-functional indicator gene. In certain embodiments, indicator gene is a luciferase gene.

In another aspect, the disclosure provides a packaging host cell that comprises a vector of the disclosure. In certain embodiments, the packaging host cell is a mammalian host cell. In certain embodiments, the packaging host cell is a human host cell. In certain embodiments, the packaging host cell is a human embryonic kidney cell. In certain embodiments, the packaging host cell is a 293 cell. In certain embodiments, the packaging host cell is derived from a human hepatoma cell line. In certain embodiments, the packaging host cell is a HepG2 cell. In certain embodiments, the packaging host cell is a Huh7 cell.

In another aspect, the invention provides a method for determining resistance of an HIV infecting a patient to an integrase inhibitor. In certain embodiments, the method comprises determining the susceptibility of the HIV to the integrase inhibitor according to a method of the invention, and comparing the determined susceptibility of the HIV to the integrase inhibitor with a standard curve of susceptibility of the HIV to the integrase inhibitor. In certain embodiments, a decrease in the susceptibility of the HIV to the integrase inhibitor relative to the standard curve indicates that the HIV is resistant to the integrase inhibitor. In certain embodiments, the amount of the decrease in susceptibility of the HIV to the integrase inhibitor indicates the degree to which the HIV is resistant to the integrase inhibitor.

In another aspect, the invention provides a method for determining the progression or development of resistance of an HIV infecting a patient to an integrase inhibitor. In certain embodiments, the method comprises determining the susceptibility of the HIV to the integrase inhibitor at a first time according to a method of the invention; assessing the effectiveness of the integrase inhibitor according to a method of the invention at a later second time; and comparing the effectiveness of the integrase inhibitor assessed at the first and second time. In certain embodiments, a patient-derived segment is obtained from the patient at about the first time. In certain embodiments, a decrease in the susceptibility of the HIV to the integrase inhibitor at the later second time as compared to the first time indicates development or progression of anti-viral drug resistance in the HIV infecting the patient.

In another aspect, the present invention provides a method for determining the susceptibility of an HIV infecting a patient to an integrase inhibitor. In certain embodiments of the disclosure, the method comprises culturing a host cell comprising a patient-derived segment obtained from the HIV and an indicator gene in the presence of varying concentrations of the anti-HIV drug, measuring the activity of the indicator gene in the host cell for the varying concentrations of the anti-HIV drug; and determining the IC50 of the HIV to the anti-viral drug, wherein the IC50 of the HIV to the anti-viral drug indicates the susceptibility of the HIV to the anti-HD/ drug. In certain embodiments, the activity of the indicator gene depends on the activity of a polypeptide encoded by the patient-derived segment. In certain embodiments, the patient-derived segment comprises a nucleic acid sequence that encodes integrase or RNAse H. In certain embodiments, the IC50 of the HIV can be determined by plotting the activity of the indicator gene observed versus the log of anti-HIV drug concentration.

In still another aspect, the invention provides a method for determining the susceptibility of a population of HIV infecting a patient to an integrase inhibitor. In certain embodiments, the method comprises culturing a host cell comprising a plurality of patient-derived segments from the HIV population and an indicator gene in the presence of the integrase inhibitor , measuring the activity of the indicator gene in the host cell; and comparing the activity of the indicator gene as measured with a reference activity of the indicator gene, wherein the difference between the measured activity of the indicator gene relative to the reference activity con⁻elates with the susceptibility of the HIV to the integrase inhibitor , thereby determining the susceptibility of the HIV to the integrase inhibitor . In certain embodiments, the activity of the indicator gene depends on the activity of a plurality of polypeptidse encoded by the plurality of patient-derived segments. In certain embodiments, the patient-derived segment comprises a nucleic acid sequence that encodes integrase or RNAse H. In certain embodiments, the plurality of patient-derived segments is prepared by amplifying the patient-derived segments from a plurality of nucleic acids obtained from a sample from the patient.

In yet another aspect, the present disclosure provides a method for determining the susceptibility of a population of HIV infecting a patient to an anti-HIV drug. In certain embodiments, the method comprises culturing a host cell comprising a plurality of patient-derived segments obtained from the population of HIV and an indicator gene in the presence of varying concentrations of the anti-HIV drug, measuring the activity of the indicator gene in the host cell for the varying concentrations of the anti-HIV drug; and determining the IC₅₀ of the population of HIV to the anti-viral drug, wherein the IC50 of the population of HIV to the anti-viral drug indicates the susceptibility of the population of HIV to the anti-HIV drug. In certain embodiments, the host cell comprises a patient-derived segment and an indicator gene. In certain embodiments, the activity of the indicator gene depends on the activity of a plurality of polypeptides encoded by the plurality of patient-derived segments. In certain embodiments, the plurality of patient-derived segments comprises a nucleic acid sequence that encodes integrase or RNAse H. In certain embodiments, the IC₅₀ of the population of HIV can be determined by plotting the activity of the indicator gene observed versus the log of anti-HIV drug concentration. In certain embodiments, the plurality of patient-derived segments is prepared by amplifying the patient-derived segments from a plurality of nucleic acids obtained from a sample from the patient.

### 5.3.1. Construction of a Resistance Test Vector

In certain embodiments, the resistance test vector can be made by insertion of a patient-derived segment into an indicator gene viral vector. Generally, in such embodiments, the resistance test vectors do not comprise all genes necessary to produce a fully infectious viral particle. In certain embodiments, the resistance test vector can be made by insertion of a patient-derived segment into a packaging vector while the indicator gene is contained in a second vector, for example an indicator gene viral vector. In certain embodiments, the resistance test vector can be made by insertion of a patient-derived segment into a packaging vector while the indicator gene is integrated into the genome of the host cell to be infected with the resistance test vector.

If a drug were to target more than one functional viral sequence or viral gene product, patient-derived segments comprising each functional viral sequence or viral gene product can be introduced into the resistance test vector. In the case of combination therapy, where two or more anti-HIV drugs targeting the same or two or more different functional viral sequences or viral gene products are being evaluated, patient-derived segments comprising each such functional viral sequence or viral gene product can be inserted in the resistance test vector. The patient-derived segments can be inserted into unique restriction sites or specified locations, called patient sequence acceptor sites, in the indicator gene viral vector or for example, a packaging vector depending on the particular construction selected

Patient-derived segments can be incorporated into resistance test vectors using any of suitable cloning technique known by one of skill in the art without limitation. For example, cloning via the introduction of class II restriction sites into both the plasmid backbone and the patient-derived segments, which is preferred, or by uracil DNA glycosylase primer cloning.

The patient-derived segment may be obtained by any method of molecular cloning or gene amplification, or modifications thereof, by introducing patient sequence acceptor sites, as described below, at the ends of the patient-derived segment to be introduced into the resistance test vector. In a preferred embodiment, a gene amplification method such as PCR can be used to incorporate restriction sites corresponding to the patient-sequence acceptor sites at the ends of the primers used in the PCR reaction. Similarly, in a molecular cloning method such as cDNA cloning, the restriction sites can be incorporated at the ends of the primers used for first or second strand cDNA synthesis, or in a method such as primer-repair of DNA, whether cloned or uncloned DNA, the restriction sites can be incorporated into the primers used for the repair reaction. The patient sequence acceptor sites and primers can be designed to improve the representation of patient-derived segments. Sets of resistance test vectors having designed patient sequence acceptor sites allows representation of patient-derived segments that could be underrepresented in one resistance test vector alone.

Resistance test vectors can be prepared by modifying an indicator gene viral vector by introducing patient sequence acceptor sites, amplifying or cloning patient-derived segments and introducing the amplified or cloned sequences precisely into indicator gene viral vectors at the patient sequence acceptor sites. In certain embodiments, the resistance test vectors can be constructed from indicator gene viral vectors, which in turn can be derived from genomic viral vectors or subgenomic viral vectors and an indicator gene cassette, each of which is described below. Resistance test vectors can then be introduced into a host cell. Alternatively, in certain embodiments, a resistance test vector can be prepared by introducing patient sequence acceptor sites into a packaging vector, amplifying or cloning patient-derived segments and inserting the amplified or cloned sequences precisely into the packaging vector at the patient sequence acceptor sites and co-transfecting this packaging vector with an indicator gene viral vector.

In one preferred embodiment, the resistance test vector may be introduced into packaging host cells together with packaging expression vectors, as defined below, to produce resistance test vector viral particles that are used in drug resistance and susceptibility tests that are referred to herein as a "particle-based test." In an alternative embodiment, the resistance test vector may be introduced into a host cell in the absence of packaging expression vectors to carry out a drug resistance and susceptibility test that is referred to herein as a "non-particle-based test." As used herein a "packaging expression vector" provides the factors, such as packaging proteins (*e.g*., structural proteins such as core and envelope polypeptides), transacting factors, or genes required by replication-defective HIV. In such a situation, a replication-competent viral genome is enfeebled in a manner such that it cannot replicate on its own. This means that, although the packaging expression vector can produce the trans-acting or missing genes required to rescue a defective viral genome present in a cell containing the enfeebled genome, the enfeebled genome cannot rescue itself. Such embodiments are particularly useful for preparing viral particles that comprise resistance test vectors which do not comprise all viral genes necessary to produce a fully infectious viral particle.

In certain embodiments, the resistance test vectors comprise an indicator gene, though as described above, the indicator gene need not necessarily be present in the resistance test vector. Examples of indicator genes include, but are not limited to, the E. coli lacZ gene which encodes beta-galactosidase, the luc gene which encodes luciferase either from, for example, Photonis pyralis (the firefly) or Renilla reniformis (the sea pansy), the E. coli phoA gene which encodes alkaline phosphatase, green fluorescent protein and the bacterial CAT gene which encodes chloramphenicol acetyltransferase. A preferred indicator gene is firefly luciferase. Additional examples of indicator genes include, but are not limited to, secreted proteins or cell surface proteins that are readily measured by assay, such as radioimmunoassay (RIA), or fluorescent activated cell sorting (FACS), including, for example, growth factors, cytokines and cell surface antigens (e.g. growth hormone, Il-2 or CD4, respectively). Still other exemplary indicator genes include selection genes, also referred to as selectable markers. Examples of suitable selectable markers for mammalian cells are dihydrofolate reductase (DHFR), thymidine kinase, hygromycin, neomycin, zeocin or *E. coli* gpt. In the case of the foregoing examples of indicator genes, the indicator gene and the patient-derived segment are discrete, *i.e.* distinct and separate genes. In some cases, a patient-derived segment may also be used as an indicator gene. In one such embodiment in which the patient-derived segment corresponds to one or more HIV genes which is the target of an anti-HIV agent, one of the HIV genes may also serve as the indicator gene. For example, a viral protease gene may serve as an indicator gene by virtue of its ability to cleave a chromogenic substrate or its ability to activate an inactive zymogen which in turn cleaves a chromogenic substrate, giving rise in each case to a color reaction. In all of the above examples of indicator genes, the indicator gene may be either "functional" or "non-functional" but in each case the expression of the indicator gene in the target cell is ultimately dependent upon the action of the patient-derived segment. Generally, the activity of the indicator gene, *e.g*., a functional property of the indicator gene such as emission of light or generation of a chromogenic substrate, can be monitored. However, the activity of an indicator gene can also be monitored by determining the amount of expression of the indicator gene using any convenient method known by one of skill in the art.

In certain embodiments, the indicator gene may be capable of being expressed in a host cell transfected with a resistance test vector and a packaging expression vector, independent of the patient-derived segment, however the functional indicator gene can not be expressed in the target host cell, as defined below, without the production of functional resistance test vector particles and their effective infection of the target host cell. In such embodiments, the indicator gene is referred to as a "functional indicator gene." In certain embodiments, the functional indicator gene cassette, comprising control elements and a gene encoding an indicator protein, is inserted into the indicator gene viral vector with the same or opposite transcriptional orientation as the native or foreign enhancer/promoter of the viral vector.

In alternate embodiments, the indicator gene may be a "non-functional indicator gene" in that the indicator gene is not efficiently expressed in a packaging host cell transfected with the resistance test vector, until it is converted into a functional indicator gene through the action of one or more of the patient-derived segment products. An indicator gene can be rendered non-functional through genetic manipulation as described below.

In certain embodiments, an indicator gene can be rendered non-functional due to the location of the promoter, in that, although the promoter is in the same transcriptional orientation as the indicator gene, it follows rather than precedes the indicator gene coding sequence. This misplaced promoter is referred to as a "permuted promoter." In addition to the permuted promoter, the orientation of the non-functional indicator gene is opposite to that of the native or foreign promoter/enhancer of the viral vector. Thus, the coding sequence of the non-functional indicator gene can be transcribed by neither the permuted promoter nor by the viral promoters. The non-functional indicator gene and its permuted promoter can be rendered functional by the action of one or more of the viral proteins. In one example of a non-functional indicator gene with a permuted promoter, a T7 phage RNA polymerase promoter (herein referred to as T7 promoter) can be placed in the 5' LTR in the same transcriptional orientation as the indicator gene. In such embodiments, indicator gene cannot be transcribed by the T7 promoter as the indicator gene cassette is positioned upstream of the T7 promoter. The non-functional indicator gene in the resistance test vector can be converted into a functional indicator gene by reverse transcriptase upon infection of the target cells, resulting from the repositioning of the T7 promoter by copying from the 5' LTR to the 3' LTR, relative to the indicator gene coding region. Following the integration of the repaired indicator gene into the target cell chromosome by HIV integrase, a nuclear T7 RNA polymerase expressed by the target cell can transcribe the indicator gene.

A permuted promoter may be any eukaryotic or prokaryotic promoter which can be transcribed in the target host cell known to one of skill in the art without limitation. Preferably the promoter will be small in size to enable insertion in the viral genome without disturbing viral replication. More preferably, a promoter that is small in size and is capable of transcription by a single subunit RNA polymerase introduced into the target host cell, such as a bacteriophage promoter, can be used. Examples of such bacteriophage promoters and their cognate RNA polymerases include those of phages T7, T3 and Sp6. A nuclear localization sequence (NLS) may be attached to the RNA polymerase to localize expression of the RNA polymerase to the nucleus where they may be needed to transcribed the repaired indicator gene. Such an NLS may be obtained from any nuclear-transported protein such as the SV40 T antigen. If a phage RNA polymerase is employed, an internal ribosome entry site (IRES) such as the EMC virus 5' untranslated region (UTR) may be added in front of the indicator gene for translation of the transcripts which are generally uncapped. The permuted promoter itself can be introduced at any position within the 5' LTR that is copied to the 3' LTR during reverse transcription so long as LTR function is not disrupted, preferably within the U5 and R portions of the LTR, and most preferably outside of functionally important and highly conserved regions of U5 and R. Further, blocking sequences may be added at the ends of the resistance test vector should there be inappropriate expression of the non-functional indicator gene due to transfection artifacts (DNA concatenation). In the example of the permuted T7 promoter given above, such a blocking sequence may consist of a T7 transcriptional terminator, positioned to block readthrough transcription resulting from DNA concatenation, but not transcription resulting from repositioning of the permuted T7 promoter from the 5' LTR to the 3' LTR during reverse transcription.

In other embodiments of a "nonfunctional indicator gene," an indicator gene can be rendered non-functional due to the relative location of the 5' and 3' coding regions of the indicator gene, in that the 3' coding region precedes rather than follows the 5' coding region. This misplaced coding region is referred to as a "permuted coding region." The orientation of the non-functional indicator gene may be the same or opposite to that of the native or foreign promoter/enhancer of the viral vector, as mRNA coding for a functional indicator gene will be produced in the event of either orientation. The non-functional indicator gene and its permuted coding region can be rendered functional by the action of one or more of the patient-derived segment products. An example of a non-functional indicator gene with a permuted coding region places a 5' indicator gene coding region with an associated promoter in the 3'LTR U3 region and a 3'indicator gene coding region in an upstream location of the HIV genome, with each coding region having the same transcriptional orientation as the viral LTRs. The 5' and 3' coding regions may also have associated splice donor and acceptor sequences, respectively, which may be heterologous or artificial splicing signals. The indicator gene cannot be functionally transcribed either by the associated promoter or viral promoters, as the permuted coding region prevents the formation of functionally spliced transcripts. The non-functional indicator gene in the resistance test vector is converted into a functional indicator gene by reverse transcriptase upon infection of the target cells, resulting from the repositioning of the 5' and 3' indicator gene coding regions relative to one another, by copying of the 3' LTR to the 5' LTR. Following transcription by the promoter associated with the 5' coding region, RNA splicing can join the 5' and 3' coding regions to produce a functional indicator gene product.

In another embodiment of a "non-functional indicator gene," the indicator gene is rendered non-functional through use of an "inverted intron," *i.e.,* an intron inserted into the coding sequence of the indicator gene with a transcriptional orientation opposite to that of the indicator gene. The overall transcriptional orientation of the indicator gene cassette including its own linked promoter can be opposite to that of the viral control elements, while the orientation of the artificial intron can be the same as the viral control elements. Transcription of the indicator gene by its own linked promoter does not lead to the production of functional transcripts, as the inverted intron cannot be spliced in this orientation. Transcription of the indicator gene by the viral control elements does, however, lead to the removal of the inverted intron by RNA splicing, although the indicator gene is still not functionally expressed as the resulting transcript has an antisense orientation. Following the reverse transcription of this transcript and integration of the resultant retroviral DNA, the indicator gene can be functionally transcribed using its own linked promoter as the inverted intron has been previously removed. In this case, the indicator gene itself may contain its own functional promoter with the entire transcriptional unit oriented opposite to the viral control elements. Thus the non-functional indicator gene is in the wrong orientation to be transcribed by the viral control elements and it cannot be functionally transcribed by its own promoter, as the inverted intron cannot be properly excised by splicing. However, transcription by the viral promoters (HIV LTR) results in the removal of the inverted intron by splicing. As a consequence of reverse transcription of the resulting spliced transcript and the integration of the resulting provirus into the host cell chromosome, the indicator gene can now be functionally transcribed by its own promoter. The inverted intron, consisting of a splice donor and acceptor site to remove the intron, is preferably located in the coding region of the indicator gene in order to disrupt translation of the indicator gene. The splice donor and acceptor may be any splice donor and acceptor. A preferred splice donor-receptor is the CMV IE splice donor and the splice acceptor of the second exon of the human alpha globin gene ("intron A").

As discussed above, a resistance test vector can be assembled from an indicator gene viral vector. As used herein, "indicator gene viral vector" refers to a vector(s) comprising an indicator gene and its control elements and one or more viral genes. The indicator gene viral vector can be assembled from an indicator gene cassette and a "viral vector," defined below. The indicator gene viral vector may additionally include an enhancer, splicing signals, polyadenylation sequences, transcriptional terminators, or other regulatory sequences. Additionally the indicator gene in the indicator gene viral vector may be functional or nonfunctional. In the event that the viral segments which are the target of the anti-viral drug are not included in the indicator gene viral vector, they can be provided in a second vector. An "indicator gene cassette" comprises an indicator gene and control elements, and, optionally, is configured with restriction enzyme cleavage sites at its ends to facilitate introduction of the cassette into a viral vector. A "viral vector" refers to a vector comprising some or all of the following: viral genes encoding a gene product, control sequences, viral packaging sequences, and in the case of a retrovirus, integration sequences. The viral vector may additionally include one or more viral segments, one or more of which may be the target of an anti-viral drug. Two examples of a viral vector which contain viral genes are referred to herein as an "genomic viral vector" and a "subgenomic viral vector." A "genomic viral vector" is a vector which may comprise a deletion of a one or more viral genes to render the virus replication incompetent, *e.g*., unable to express all of the proteins necessary to produce a fully infectious viral particle, but which otherwise preserves the mRNA expression and processing characteristics of the complete virus. In one embodiment for an HIV drug susceptibility and resistance test, the genomic viral vector comprises the HIV *gag, pol, vif, vpr, tat, rev, vpu,* and *nef* genes. In certain embodiments, some, most or all of *env* can be deleted. A "subgenomic viral vector" refers to a vector comprising the coding region of one or more viral genes which may encode the proteins that are the target(s) of the anti-viral drug. In a preferred embodiment, a subgenomic viral vector comprises the HIV *pol* gene, or a portion thereof. Two examples of proviral clones that can be used for viral vector construction are: HXB2 (Fisher et al., 1986 Nature 320:367-371) and NL4-3 (Adachi et al., 1986, J. Virol., 59:284-291). In certain embodiments, the viral coding genes can be under the control of a native enhancer/promoter. In certain embodiments, the viral coding genes can be under the control of a foreign viral or cellular enhancer/promoter. In a preferred embodiment, the genomic or subgenomic viral coding regions can be under the control of the native enhancer/promoter of the HIV-LTR U3 region or the CMV immediate-early (IE) enhancer/promoter. In certain embodiments of an indicator gene viral vector that contains one or more viral genes which are the targets or encode proteins which are the targets of one or more anti-viral drug(s), the vector can comprise patient sequence acceptor sites. The patient-derived segments can be inserted in the patient sequence acceptor site in the indicator gene viral vector which is then referred to as the resistance test vector, as described above.

"Patient sequence acceptor sites" are sites in a vector for insertion of patient-derived segments. In certain embodiments, such sites may be: 1) unique restriction sites introduced by site-directed mutagenesis into a vector; 2) naturally occurring unique restriction sites in the vector; or 3) selected sites into which a patient-derived segment may be inserted using alternative cloning methods (e.g. UDG cloning). In certain embodiments, the patient sequence acceptor site is introduced into the indicator gene viral vector by site-directed mutagenesis. The patient sequence acceptor sites can be located within or near the coding region of the viral protein which is the target of the anti-viral drug. The viral sequences used for the introduction of patient sequence acceptor sites are preferably chosen so that no change is made in the amino acid coding sequence found at that position. If a change is made in the amino acid coding sequence at the position, the change is preferably a conservative change. Preferably the patient sequence acceptor sites can be located within a relatively conserved region of the viral genome to facilitate introduction of the patient-derived segments. Alternatively, the patient sequence acceptor sites can be located between functionally important genes or regulatory sequences. Patient-sequence acceptor sites may be located at or near regions in the viral genome that are relatively conserved to permit priming by the primer used to introduce the corresponding restriction site into the patient-derived segment. To improve the representation of patient-derived segments further, such primers may be designed as degenerate pools to accommodate viral sequence heterogeneity, or may incorporate residues such as deoxyinosine (I) which have multiple base-pairing capabilities. Sets of resistance test vectors having patient sequence acceptor sites that define the same or overlapping restriction site intervals may be used together in the drug resistance and susceptibility tests to provide representation of patient-derived segments that contain internal restriction sites identical to a given patient sequence acceptor site, and would thus be underrepresented in either resistance test vector alone.

Construction of the vectors of the disclosure employs standard ligation and restriction techniques which are well understood in the art. *See,* for example, Ausubel et al., 2005, Current Protocols in Molecular Biology Wiley--Interscience and Sambrook et al., 2001, Molecular Cloning: A Laboratory Manual, Cold Spring Harbor Laboratory, N.Y.. Isolated plasmids, DNA sequences, or synthesized oligonucleotides can be cleaved, tailored, and relegated in the form desired. The sequences of all DNA constructs incorporating synthetic DNA can be confirmed by DNA sequence analysis. *See,* for example, Sanger et al., 1977, P.N.A.S. USA 74:5463-5467.

In addition to the elements discussed above, the vectors used herein may also contain a selection gene, also termed a selectable marker. In certain embodiments, the selection gene encodes a protein, necessary for the survival or growth of a host cell transformed with the vector. Examples of suitable selectable markers for mammalian cells include the dihydrofolate reductase gene (DHFR), the ornithine decarboxylase gene, the multi-drug resistance gene (mdr), the adenosine deaminase gene, and the glutamine synthase gene. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. The second category is referred to as dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin (*see* Southern and Berg, 1982, J. Molec. Appl. Genet. 1:327, mycophenolic acid (*see* Mulligan and Berg, 1980, Science 209:1422, or hygromycin (*see* Sugden et al., 1985, Mol. Cell. Biol. 5:410-413. The three examples given above employ bacterial genes under eukaryotic control to convey resistance to the appropriate drug neomycin (G418 or genticin), xgpt (mycophenolic acid) or hygromycin, respectively.

### 5.3.2. Host Cells

In certain embodiments, the methods of the invention comprise culturing a host cell that comprises a patient-derived segment and an indicator gene. In certain embodiments, the host cells can be mammalian cells. Preferred host cells can be derived from human tissues and cells which are the principle targets of viral infection. Such host cells include, but are not limited to, human cells such as human T cells, monocytes, macrophage, dendritic cells, Langerhans cells, hematopoeitic stem cells or precursor cells, and the like. Human-derived host cells allow the anti-viral drug to enter the cell efficiently and be converted by the cellular enzymatic machinery into the metabolically relevant form of the anti-viral inhibitor. In some embodiments, host cells can be referred to herein as a "packaging host cells," "resistance test vector host cells," or "target host cells." A "packaging host cell" refers to a host cell that provides the transacting factors and viral packaging proteins required by the replication defective viral vectors used herein, such as, *e.g.,* the resistance test vectors, to produce resistance test vector viral particles. The packaging proteins may provide for expression of viral genes contained within the resistance test vector itself, a packaging expression vector(s), or both. A packaging host cell can be a host cell which is transfected with one or more packaging expression vectors and when transfected with a resistance test vector is then referred to herein as a "resistance test vector host cell" and is sometimes referred to as a packaging host cell/resistance test vector host cell. Preferred host cells for use as packaging host cells include 293 human embryonic kidney cells (Graham et al., 1977, J. Gen Virol. 36:59), BOSC23 (Pear et al., 1993, P.N.A.S. USA. 90:8392), and tsa54 and tsa201 cell lines (Heinzel et al., 1988, J. Virol. 62:3738). A "target host cell" refers to a cell to be infected by resistance test vector viral particles produced by the resistance test vector host cell in which expression or inhibition of the indicator gene takes place. Preferred host cells for use as target host cells include human T cell leukemia cell lines including Jurkat (ATCC T1B-152), H9 (ATCC HTB-176), CEM (ATCC CCL-119), HUT78 (ATCC T1B-161), and derivatives thereof; and 293 cells.

Unless otherwise provided, the method used herein for transformation of the host cells is the calcium phosphate co-precipitation method of Graham and van der Eb, 1973, Virology 52:456-457. Alternative methods for transfection include, but are not limited to, electroporation, the DEAE-dextran method, lipofection and biolistics. *See, e.g.,* Kriegler, 1990, Gene Transfer and Expression: A Laboratory Manual, Stockton Press.

Host cells may be transfected with the expression vectors of the present disclosure and cultured in conventional nutrient media modified as is appropriate for inducing promoters, selecting transformants or amplifying genes. Host cells are cultured in F12: DMEM (Gibco) 50:50 with added glutamine and without antibiotics. The culture conditions, such as temperature, pH and the like, are those previously used with the host cell selected for expression, and will be apparent to the ordinarily skilled artisan.

### 4.2.1. Drug Susceptibility and Resistance Tests

Drug susceptibility and resistance tests may be carried out in one or more host cells. Viral drug susceptibility is determined as the concentration of the anti-viral agent at which a given percentage of indicator gene expression is inhibited *(e.g*., the IC₅0 for an anti-viral agent is the concentration at which 50% of indicator gene expression is inhibited). A standard curve for drug susceptibility of a given anti-viral drug can be developed for a viral segment that is either a standard laboratory viral segment or from a drug-naive patient (i.e. a patient who has not received any anti-viral drug) using the method of this invention. Correspondingly, viral drug resistance can be determined by detecting a decrease in viral drug susceptibility for a given patient either by comparing the drug susceptibility to such a given standard or by making sequential measurement in the same patient over time, as determined by increased inhibition of indicator gene expression (i.e. decreased indicator gene expression).

In certain embodiments, resistance test vector viral particles are produced by a first host cell (the resistance test vector host cell) that is prepared by transfecting a packaging host cell with the resistance test vector and packaging expression vector(s). The resistance test vector viral particles can then be used to infect a second host cell (the target host cell) in which the expression of the indicator gene is measured. Such a two cell system comprising a packaging host cell which is transfected with a resistance test vector, which is then referred to as a resistance test vector host cell, and a target cell are used in the case of either a functional or non-functional indicator gene. Functional indicator genes are efficiently expressed upon transfection of the packaging host cell, and thus infection of a target host cell with resistance test vector host cell supernatant is needed to accurately determine drug susceptibility. Non-functional indicator genes with a permuted promoter, a permuted coding region, or an inverted intron are not efficiently expressed upon transfection of the packaging host cell and thus the infection of the target host cell can be achieved either by co-cultivation by the resistance test vector host cell and the target host cell or through infection of the target host cell using the resistance test vector host cell supernatant. In the second type of drug susceptibility and resistance test, a single host cell (the resistance test vector host cell) also serves as a target host cell. The packaging host cells are transfected and produce resistance test vector viral particles and some of the packaging host cells also become the target of infection by the resistance test vector particles. Drug susceptibility and resistance tests employing a single host cell type are possible with viral resistance test vectors comprising a non-functional indicator gene with a permuted promoter, a permuted coding region, or an inverted intron. Such indicator genes are not efficiently expressed upon transfection of a first cell, but are only efficiently expressed upon infection of a second cell, and thus provide an opportunity to measure the effect of the anti-viral agent under evaluation. In the case of a drug susceptibility and resistance test using a resistance test vector comprising a functional indicator gene, neither the co-cultivation procedure nor the resistance and susceptibility test using a single cell type can be used for the infection of target cells. A resistance test vector comprising a functional indicator gene can use a two cell system using filtered supernatants from the resistance test vector host cells to infect the target host cell.

In certain embodiments, a particle-based resistance tests can be carried out with resistance test vectors derived from genomic viral vectors, *e.g*., pHIVΔ1ucRHIN or pHIVΔ1ucPOL, which can be cotransfected with the packaging expression vector pVLenv4070A (also referred to as pCXAS-4070Aenv). Alternatively, a particle-based resistance test may be carried out with resistance test vectors derived from subgenomic viral vectors which are cotransfected with the packaging expression vector pVL-env4070 and either PLTR-HIV3' or pCMV-HIV3'. In another embodiment of the disclosure, non-particle-based resistance tests can be carried out using each of the above described resistance test vectors by transfection of selected host cells in the absence of packaging expression vectors.

In the case of the particle-based susceptibility and resistance test, resistance test vector viral particles can be produced by a first host cell (the resistance test vector host cell), that can be prepared by transfecting a packaging host cell with the resistance test vector and packaging expression vector(s) as described above. The resistance test vector viral particles can then be used to infect a second host cell (the target host cell) in which the expression of the indicator gene is measured. In a second type of particle-based susceptibility and resistance test, a single host cell type (the resistance test vector host cell) serves both purposes: some of the packaging host cells in a given culture can be transfected and produce resistance test vector viral particles and some of the host cells in the same culture can be the target of infection by the resistance test vector particles thus produced. Resistance tests employing a single host cell type are possible with resistance test vectors comprising a non-functional indicator gene with a permuted promoter since such indicator genes can be efficiently expressed upon infection of a permissive host cell, but are not efficiently expressed upon transfection of the same host cell type, and thus provide an opportunity to measure the effect of the anti-viral agent under evaluation. For similar reasons, resistance tests employing two cell types may be carried out by co-cultivating the two cell types as an alternative to infecting the second cell type with viral particles obtained from the supernatants of the first cell type.

In the case of the non-particle-based susceptibility and resistance test, resistance tests can be performed by transfection of a single host cell with the resistance test vector in the absence of packaging expression vectors. Non-particle based resistance tests can be carried out using the resistance test vectors comprising non-functional indicator genes with either permuted promoters, permuted coding regions or inverted introns. These non-particle based resistance tests are performed by transfection of a single host cell type with each resistance test vector in the absence of packaging expression vectors. Although the non-functional indicator genes contained within these resistance test vectors are not efficiently expressed upon transfection of the host cells, there is detectable indicator gene expression resulting from non-viral particle-based reverse transcription. Reverse transcription and strand transfer results in the conversion of the permuted, non-functional indicator gene to a non-permuted, functional indicator gene. As reverse transcription is completely dependent upon the expression of the *pol* gene contained within each resistance test vector, anti-viral agents may be tested for their ability to inhibit the *pol* gene products, including, for example, reverse transcriptase, RNAse H, or integrase, encoded by the patient-derived segments contained within the resistance test vectors. As such, embodiments where the patient-derived segment comprises the entire *pol* gene are appropriate for this kind of assay. Reverse transcription and strand transfer results in the conversion of the non-functional indicator gene to a functional indicator gene. As reverse transcription depends upon the expression of the genes encoded by the patient-derived segment contained within each resistance test vector, anti-viral agents may be tested for their ability to inhibit the gene products encoded by the patient-derived segments contained within the resistance test vectors.

The packaging host cells can be transfected with the resistance test vector and the appropriate packaging expression vector(s) to produce resistance test vector host cells. In certain embodiments, individual anti-viral agents, including reverse transcriptase inhibitors such as AZT, ddI, ddC, d4T, 3TC, EFV, DLV, NVP, and the like, or integrase inhibitors such as L-870,810 or L-731,988, as well as combinations thereof, can be added to individual plates of packaging host cells at the time of their transfection, at an appropriate range of concentrations. Twenty-four to 48 hours after transfection, target host cells can be infected by co-cultivation with resistance test vector host cells or with resistance test vector viral particles obtained from filtered supernatants of resistance test vector host cells. Each anti-viral agent, or combination thereof, can be added to the target host cells prior to or at the time of infection to achieve the same final concentration of the given agent, or agents, present during the transfection. In other embodiments, the anti-viral agent(s) can be omitted from the packaging host cell culture, and added only to the target host cells prior to or at the time of infection.

Determination of the expression or inhibition of the indicator gene in the target host cells infected by co-cultivation or with filtered viral supernatants can be performed measuring indicator gene expression or activity. For example, in the case where the indicator gene is the firefly luc gene, luciferase activity can be measured. The reduction in luciferase activity observed for target host cells infected with a given preparation of resistance test vector viral particles in the presence of a given antiviral agent, or agents, as compared to a control run in the absence of the antiviral agent, generally relates to the log of the concentration of the antiviral agent as a sigmoidal curve. This inhibition curve can be used to calculate the apparent inhibitory concentration (IC) of that agent, or combination of agents, for the viral target product encoded by the patient-derived segments present in the resistance test vector.

In the case of a one cell susceptibility and resistance test, host cells can be transfected with the resistance test vector and the appropriate packaging expression vector(s) to produce resistance test vector host cells. Individual antiviral agents, or combinations thereof, can be added to individual plates of transfected cells at the time of their transfection, at an appropriate range of concentrations. Twenty-four to 72 hours after transfection, cells can be collected and assayed for indicator gene, e.g., firefly luciferase, activity. As transfected cells in the culture do not efficiently express the indicator gene, transfected cells in the culture, as well superinfected cells in the culture, can serve as target host cells for indicator gene expression. The reduction in luciferase activity observed for cells transfected in the presence of a given antiviral agent, or agents as compared to a control run in the absence of the antiviral agent(s), generally relates to the log of the concentration of the antiviral agent as a sigmoidal curve. This inhibition curve can be used to calculate the apparent inhibitory concentration (IC) of an agent, or combination of agents, for the viral target product encoded by the patient-derived segments present in the resistance test vector.

### 5.3.4. Antiviral Drugs/Drug Candidates

The antiviral drugs being added to the test system can be added at selected times depending upon the target of the antiviral drug. HIV reverse transcriptase inhibitors, including AZT, ddI, ddC, d4T, 3TC, efavirenz, delaviridine, nevaripine, and the like, and HIV integrase, such as L-731,988 or L-870,810, or RNAse H inhibitors, such as RDS 1643, can be added to individual plates of target host cells at the time of infection by the resistance test vector viral particles, at a test concentration. Alternatively, the antiviral drugs may be present throughout the assay. The test concentration is selected from a range of concentrations which is typically between about 0.1 nM and about 100 *µ*M, between about 1 nM and about 100 *µ*M, between about 10 nM and about 100 *µ*M, between about 0.1 nM and about 10 *µ*M, between about 1 nM and about 10 *µ*M, between about 10 nM and about 10 *µ*M*,* between about 0.1 nM and about 1 *µ*M, between about 1 nM and about 1 *µ*M, or between about 0.01nM and about 0.1 *µ*M*.*

Further guidance on RNAse H inhibitors and integrase inhibitors that can be used in the methods of the invention may be found in, for example, Tramontano et al., 2005, Antiviral Res. 65:117-24; Andreola, 2004, Curr Pharm Des 10:3713-23; Hang et al., 2004, Biochem Biophys Res Commun 317:321-9; Skillman et al., 2002, Bioorg Chem 30:443-58; Dayam et al., 2005, J Med Chem. 48:111-20; Turpin, 2003, Expert Rev Anti Infect Ther 1:97-128; Sechi et al., 2004, JMed Chem 47:5298-310; Middleton et al., 2004, Antiviral Res 64:35-45; Boyle, 2004, AIDS Read 14:412-6, 452; Witvrouw et al., 2004, Curr Drug Metab. 5:291-304; Reinke et al., 2004, Virology 326:203-19; and Johnson et al., 2004, Curr Top Med Chem 4:1059-77.

In certain embodiments, a candidate antiviral compound can be tested in a drug susceptibility test of the disclosure. The candidate antiviral compound can be added to the test system at an appropriate concentration and at selected times depending upon the protein target of the candidate anti-viral. Alternatively, more than one candidate antiviral compound may be tested or a candidate antiviral compound may be tested in combination with an approved antiviral drug such as AZT, ddI, ddC, d4T, 3TC, saquinavir, ritonavir, indinavir, and the like, or a compound which is undergoing clinical trials such as, for example, L-870,810. The effectiveness of the candidate antiviral compound can be evaluated by measuring the activity of the indicator gene. If the candidate compound is effective at inhibiting a viral polypeptide activity, the activity of the indicator gene will be reduced in the presence of the candidate compound relative to the activity observed in the absence of the candidate compound. In another aspect of this embodiment, the drug susceptibility and resistance test may be used to screen for viral mutants. Following the identification of resistant mutants to either known anti-viral drugs or candidate anti-viral drugs the resistant mutants can be isolated and the DNA analyzed. A library of viral resistant mutants can thus be assembled enabling the screening of candidate anti-viral agents, either alone or in combination with other known or putative anti-viral agents.

### 4.3. Methods of Determining Replication Capacity of an HIV

In another aspect, the disclosure provides a method for determining the replication capacity of a human immunodeficiency virus (HIV). In certain embodiments, the method comprises culturing a host cell comprising a patient-derived segment and an indicator gene, measuring the activity of the indicator gene in the host cell, wherein the activity of the indicator gene between the activity of the indicator gene measured in step (b) relative to a reference activity indicates the replication capacity of the HIV, thereby determining the replication capacity of the HIV. In certain embodiments, the activity of the indicator gene depends on the activity of a polypeptide encoded by the patient-derived segment. In certain embodiments, the patient-derived segment comprises a nucleic acid sequence that encodes integrase or RNAse H.

In certain embodiments, the reference activity of the indicator gene is an amount of activity determined by performing a method of the disclosure with a standard laboratory viral segment. In certain embodiments, the standard laboratory viral segment comprises a nucleic acid sequence from HIV strain NL4-3. In certain embodiments, the standard laboratory viral segment comprises a nucleic acid sequence from HIV strain IIIB.

In certain embodiments, the HIV is determined to have increased replication capacity relative to the reference. In certain embodiments, the HIV is determined to have reduced replication capacity relative to the reference. In certain embodiments, the host cell is a 293 cell. In certain embodiments, the patient-derived segment encodes integrase. In certain embodiments, the patient-derived segment encodes RNAse H.

In certain embodiments, the phenotypic analysis can be performed using recombinant virus assays ("RVAs"). In certain embodiments, RVAs use virus stocks generated by homologous recombination or between viral vectors and viral gene sequences, amplified from the patient virus. In certain embodiments, RVAs virus stocks generated by ligating viral gene sequences, amplified from patient virus, into viral vectors. In certain embodiments, the viral vector is a HIV vector and the viral gene sequences comprise *pol* sequences, or a portion thereof. In certain embodiments, the viral gene sequences encode reverse transcriptase. In certain embodiments, the viral gene sequences encode integrase. In certain embodiments, the viral gene sequences encode the RNAse H portion of reverse transcriptase. In certain embodiments, the viral gene sequences encode reverse transcriptase and integrase. In certain embodiments, the viral gene sequences encode the RNAse H portion of reverse transcriptase and integrase.

The methods of determining replication capacity can be used, for example, with nucleic acids from amplified viral gene sequences. As discussed below, the nucleic acid can be amplified from any sample known by one of skill in the art to contain a viral gene sequence, without limitation. For example, the sample can be a sample from a human or an animal infected with the virus or a sample from a culture of viral cells. In certain embodiments, the viral sample comprises a genetically modified laboratory strain. In certain embodiments, the genetically modified laboratory strain comprises a site-directed mutation. In other embodiments, the viral sample comprises a wild-type isolate. In certain embodiments, the wild-type isolate is obtained from a treatment-naive patient. In certain embodiments, the wild-type isolate is obtained from a treatment-experienced patient.

A resistance test vector ("RTV") can then be constructed by incorporating the amplified viral gene sequences into a replication defective viral vector by using any method known in the art of incorporating gene sequences into a vector. In one embodiment, restrictions enzymes and conventional cloning methods are used. *See* Sambrook et al., 2001, Molecular Cloning: A Laboratory Manual, Cold Spring Harbor Laboratory, 3rd ed., NY; and Ausubel et al., 1989, Current Protocols in Molecular Biology, Greene Publishing Associates and Wiley Interscience, NY. In a preferred embodiment, *Apa*I*, Pin*AI*,* and *XhoI* restriction enzymes are used. Preferably, the replication defective viral vector is the indicator gene viral vector ("IGVV"). In a preferred embodiment, the viral vector contains a means for detecting replication of the RTV. Preferably, the viral vector comprises a luciferase gene.

The assay can be performed by first co-transfecting host cells with RTV DNA and a plasmid that expresses the envelope proteins of another retrovirus, for example, amphotropic murine leukemia virus (MLV). Following transfection, viral particles can be harvested from the cell culture and used to infect fresh target cells in the presence of varying amounts of anti-viral drug(s). The completion of a single round of viral replication in the fresh target cells can be detected by the means for detecting replication contained in the vector. In a preferred embodiment, the means for detecting replication is an indicator gene. In a preferred embodiment, the indicator gene is firefly luciferase. In such preferred embodiments, the completion of a single round of viral replication results in the production of luciferase.

In certain embodiments, the HIV strain that is evaluated is a wild-type isolate of HIV. In other embodiments, the HIV strain that is evaluated is a mutant strain of HIV. In certain embodiments, such mutants can be isolated from patients. In other embodiments, the mutants can be constructed by site-directed mutagenesis or other equivalent techniques known to one of skill in the art. In still other embodiments, the mutants can be isolated from cell culture. The cultures can comprise multiple passages through cell culture in the presence of antiviral compounds to select for mutations that accumulate in culture in the presence of such compounds. In certain embodiments, the antiviral compounds can be L-870,810 or L-731,988.

In one embodiment, viral nucleic acid, for example, HIV-1 RNA is extracted from plasma samples, and a fragment of, or entire viral genes can be amplified by methods such as, but not limited to PCR. *See, e.g.,* Hertogs et al., 1998, Antimicrob Agents Chemother 42(2):269-76. In one example, a 1.8-kb fragment containing the portion of HIV RT corresponding to RNAse H and integrase coding sequence can be amplified by reverse transcription-PCR. In another example, a 3.3-kb fragment containing the entire RT and integrase coding sequence can be amplified by reverse transcription-PCR. The pool of amplified nucleic acid, for example, the RH-IN-coding sequences, can then be cotransfected into a host cell such as CD4⁺ T lymphocytes (MT4) with the a plasmid from which most of the RH-IN sequences are deleted. Homologous recombination can then lead to the generation of chimeric viruses containing viral coding sequences, such as the RH- and IN-coding sequences derived from HIV RNA in plasma. The replication capacities of the chimeric viruses can be determined by any cell viability assay known in the art, and compared to replication capacities of a reference to assess whether a virus has altered replication capacity or is resistant or hypersusceptible to the antiviral drug. In certain embodiments, the reference can be the replication capacities of a statistically significant number of individual viral isolates. In other embodiments, the reference can be the replication capacity of a reference virus such as NL4-3 or IIIB. For example, an MT4 cell-3-(4,5-dimethylthiazol-2-yl) -2,5-diphenyltetrazolium bromide-based cell viability assay can be used in an automated system that allows high sample throughput.

Other assays for evaluating the phenotypic susceptibility of a virus to anti-viral drugs known to one of skill in the art can be adapted to determine replication capacity or to determine antiviral drug susceptibility or resistance. *See, e.g.,* Shi and Mellors, 1997, Antimicrob Agents Chemother. 41(12):2781-85; Gervaix et al., 1997, Proc Natl Acad Sci U. S. A. 94(9):4653-8; Race et al., 1999, AIDS 13:2061-2068, according to the method of the present invention.

One skilled in the art will recognize that the above-described methods for determining the replication capacity of an HIV can readily be adapted to perform methods for determining anti-HIV drug susceptibility. Similarly, one of skill in the art will recognize that the above-described methods for determining anti-HIV drug susceptibility can readily be adapted to perform methods for determining the replication capacity of an HIV. Adaptation of the methods for determining replication capacity can generally comprise performing the methods of the disclosure in the presence of varying concentration of antiviral drug. By doing so, the susceptibility of the HIV to the antiviral drug can be determined. Similarly, performing a method for determining anti-HIV drug susceptibility in the absence of any antiviral drug can provide a measure of the replication capacity of the HIV used in the method.

### 5.4.1. Detecting the Presence or Absence of Mutations in a Virus

The presence or absence of an mutation in a virus can be determined by any means known in the art for detecting a mutation. The mutation can be detected in the viral gene that encodes a particular protein, or in the protein itself, *i.e.,* in the amino acid sequence of the protein.

In one embodiment, the mutation is in the viral genome. Such a mutation can be in, for example, a gene encoding a viral protein, in a genetic element such as a *cis* or *trans* acting regulatory sequence of a gene encoding a viral protein, an intergenic sequence, or an intron sequence. The mutation can affect any aspect of the structure, function, replication or environment of the virus that changes its susceptibility to an anti-viral treatment and/or its replication capacity. In one embodiment, the mutation is in a gene encoding a viral protein that is the target of an currently available anti-viral treatment. In other embodiments, the mutation is in a gene or other genetic element that is not the target of a currently-available anti-viral treatment.

A mutation within a viral gene can be detected by utilizing any suitable technique known to one of skill in the art without limitation. Viral DNA or RNA can be used as the starting point for such assay techniques, and may be isolated according to standard procedures which are well known to those of skill in the art.

The detection of a mutation in specific nucleic acid sequences, such as in a particular region of a viral gene, can be accomplished by a variety of methods including, but not limited to, restriction-fragment-length-polymorphism detection based on allele-specific restriction-endonuclease cleavage (Kan and Dozy, 1978, Lancet ii:910-912), mismatch-repair detection (Faham and Cox, 1995, Genome Res 5:474-482), binding of MutS protein (Wagner et al., 1995, Nucl Acids Res 23:3944-3948), denaturing-gradient gel electrophoresis (Fisher et al., 1983, Proc. Natl. Acad. Sci. U.S.A. 80:1579-83), single-strand-conformation-polymorphism detection (Orita et al., 1983, Genomics 5:874-879), RNAase cleavage at mismatched base-pairs (Myers et al., 1985, Science 230:1242), chemical (Cotton et al., 1988, Proc. Natl. Acad. Sci. U.S.A. 85:4397-4401) or enzymatic (Youil et al., 1995, Pro. Natl. Acad. Sci. U.S.A. 92:87-91) cleavage of heteroduplex DNA, methods based on oligonucleotide-specific primer extension (Syvänen et al., 1990, Genomics 8:684-692), genetic bit analysis (Nikiforov et al., 1994, Nucl Acids Res 22:4167-4175), oligonucleotide-ligation assay (Landegren et al., 1988, Science 241:1077), oligonucleotide-specific ligation chain reaction ("LCR") (Barrany, 1991, Proc. Natl. Acad. Sci. U.S.A. 88:189-193), gap-LCR (Abravaya et al., 1995, Nucl Acids Res 23:675-682), radioactive or fluorescent DNA sequencing using standard procedures well known in the art, and peptide nucleic acid (PNA) assays (Orum et al., 1993, Nucl. Acids Res. 21:5332-5356; Thiede et al., 1996, Nucl. Acids Res. 24:983-984).

In addition, viral DNA or RNA may be used in hybridization or amplification assays to detect abnormalities involving gene structure, including point mutations, insertions, deletions and genomic rearrangements. Such assays may include, but are not limited to, Southern analyses (Southern, 1975, J. Mol. Biol. 98:503-517), single stranded conformational polymorphism analyses (SSCP) (Orita et al., 1989, Proc. Natl. Acad. Sci. USA 86:2766-2770), and PCR analyses (U.S. Patent Nos. 4,683,202; 4,683,195; 4,800,159; and 4,965,188; PCR Strategies, 1995 Innis et al. (eds.), Academic Press, Inc.).

Such diagnostic methods for the detection of a gene-specific mutation can involve for example, contacting and incubating the viral nucleic acids with one or more labeled nucleic acid reagents including recombinant DNA molecules, cloned genes or degenerate variants thereof, under conditions favorable for the specific annealing of these reagents to their complementary sequences. Preferably, the lengths of these nucleic acid reagents are at least 15 to 30 nucleotides. After incubation, all non-annealed nucleic acids are removed from the nucleic acid molecule hybrid. The presence of nucleic acids which have hybridized, if any such molecules exist, is then detected. Using such a detection scheme, the nucleic acid from the virus can be immobilized, for example, to a solid support such as a membrane, or a plastic surface such as that on a microtiter plate or polystyrene beads. In this case, after incubation, non-annealed, labeled nucleic acid reagents of the type described above are easily removed. Detection of the remaining, annealed, labeled nucleic acid reagents is accomplished using standard techniques well-known to those in the art. The gene sequences to which the nucleic acid reagents have annealed can be compared to the annealing pattern expected from a normal gene sequence in order to determine whether a gene mutation is present.

These techniques can easily be adapted to provide high-throughput methods for detecting mutations in viral genomes. For example, a gene array from Affymetrix (Affymetrix, Inc., Sunnyvale, CA) can be used to rapidly identify genotypes of a large number of individual viruses. Affymetrix gene arrays, and methods of making and using such arrays, are described in, for example, U.S. Patent Nos. 6,551,784, 6,548,257, 6,505,125, 6,489,114, 6,451,536, 6,410,229, 6,391,550, 6,379,895, 6,355,432, 6,342,355, 6,333,155, 6,308,170, 6,291,183, 6,287,850, 6,261,776, 6,225,625, 6,197,506, 6,168,948, 6,156,501, 6,141,096, 6,040,138, 6,022,963, 5,919,523, 5,837,832, 5,744,305, 5,834,758, and 5,631,734.

In addition, Ausubel et al., eds., Current Protocols in Molecular Biology, 2002, Vol. 4, Unit 25B, Ch. 22, provides further guidance on construction and use of a gene array for determining the genotypes of a large number of viral isolates. Finally, U.S. Patent Nos. 6,670,124; 6,617,112; 6,309,823; 6,284,465; and 5,723,320, describe related array technologies that can readily be adapted for rapid identification of a large number of viral genotypes by one of skill in the art.

Alternative diagnostic methods for the detection of gene specific nucleic acid molecules may involve their amplification, e.g., by PCR (U.S. Patent Nos. 4,683,202; 4,683,195; 4,800,159; and 4,965,188; PCR Strategies, 1995 Innis et al. (eds.), Academic Press, Inc.), followed by the detection of the amplified molecules using techniques well known to those of skill in the art. The resulting amplified sequences can be compared to those which would be expected if the nucleic acid being amplified contained only normal copies of the respective gene in order to determine whether a gene mutation exists.

Additionally, the nucleic acid can be sequenced by any sequencing method known in the art. For example, the viral DNA can be sequenced by the dideoxy method of Sanger et al., 1977, Proc. Natl. Acad. Sci. USA 74:5463, as further described by Messing et al., 1981, Nuc. Acids Res. 9:309, or by the method of Maxam et al., 1980, Methods in Enzymology 65:499. See also the techniques described in Sambrook et al., 2001, Molecular Cloning: A Laboratory Manual, Cold Spring Harbor Laboratory, 3rd ed., NY; and Ausubel et al., 1989, Current Protocols in Molecular Biology, Greene Publishing Associates and Wiley Interscience, NY.

Antibodies directed against the viral gene products, *i*.*e*., viral proteins or viral peptide fragments can also be used to detect mutations in the viral proteins. Alternatively, the viral protein or peptide fragments of interest can be sequenced by any sequencing method known in the art in order to yield the amino acid sequence of the protein of interest. An example of such a method is the Edman degradation method which can be used to sequence small proteins or polypeptides. Larger proteins can be initially cleaved by chemical or enzymatic reagents known in the art, for example, cyanogen bromide, hydroxylamine, trypsin or chymotrypsin, and then sequenced by the Edman degradation method.

### 4.4. Computer-Implemented Methods for Determining Anti-HIV Drug Susceptibility or Replication Capacity

In another aspect, the present disclosure provides computer-implemented methods for determining the susceptibility of an HIV to an anti-HIV drug or determining the replication capacity of an HIV. In such embodiments, the methods of the disclosure are adapted to take advantage of the processing power of modern computers. One of skill in the art can readily adapt the methods in such a manner.

In certain embodiments, the disclosure provides a computer-implemented method for determining the susceptibility of an HIV to an anti-HIV drug. In certain embodiments, the method comprises inputting information regarding the activity of an indicator gene determined according to a method of the disclosure and a reference activity of an indicator gene and instructions to compare the activity of the indicator gene determined according to a method of the disclosure with the reference activity of the indicator gene into a computer memory; and comparing the activity of the indicator gene determined according to a method of the disclosure with the reference activity of the indicator gene in the computer memory, wherein the difference between the measured activity of the indicator gene relative to the reference activity correlates with the susceptibility of the HIV to the anti-HIV drug, thereby determining the susceptibility of the HIV to the anti-HIV drug.

In certain embodiments, the methods further comprise displaying the susceptibility of the HIV to the anti-HIV drug on a display of the computer. In certain embodiments, the methods further comprise printing the susceptibility of the HIV to the anti-HIV drug on a paper.

In another aspect, the disclosure provides a print-out indicating the susceptibility of the HIV to the anti-HIV drug determined according to a method of the disclosure. In still another aspect, the disclosure provides a computer-readable medium comprising data indicating the susceptibility of the HIV to the anti-HIV drug determined according to a method of the disclosure.

In another aspect, the disclosure provides a computer-implemented method for determining the replication capacity of an HIV. In certain embodiments, the method comprises inputting information regarding the activity of an indicator gene determined according to a method of the disclosure and a reference activity of an indicator gene and instructions to compare the activity of the indicator gene determined according to a method of the disclosure with the reference activity of the indicator gene into a computer memory; and comparing the activity of the indicator gene determined according to a method of the disclosure with the reference activity of the indicator gene in the computer memory, wherein the comparison of the measured activity of the indicator gene relative to the reference activity indicates the replication capacity of the HIV, thereby determining the replication capacity of the HIV.

In certain embodiments, the methods further comprise displaying the replication capacity of the HIV on a display of the computer. In certain embodiments, the methods further comprise printing the replication capacity of the HIV on a paper.

In another aspect, the disclosure provides a print-out indicating the replication capacity of the HIV, where the replication capacity is determined according to a method of the disclosure. In still another aspect, the disclosure provides a computer-readable medium comprising data indicating the replication capacity of the HIV, where the replication capacity is determined according to a method of the disclosure.

In still another aspect, the disclosure provides an article of manufacture that comprises computer-readable instructions for performing a method of the disclosure.

In yet another aspect, the disclosure provides a computer system that is configured to perform a method of the disclosure.

### 4.5. Nucleic Acids

In another aspect, the invention provides an oligonucleotide that can conveniently be used in the preparation of patient-derived segments for use in the methods of the invention. In certain embodiments, the oligonucleotide comprises a nucleic acid sequence selected from the group consisting of SEQ ID NO:1, SEQ ID NO 2:, SEQ ID NO:3, and SEQ ID NO:4.

In certain embodiments, the nucleic acid sequence is SEQ ID NO:1. In certain embodiments, the nucleic acid sequence is SEQ ID NO:2. In certain embodiments, the nucleic acid sequence is SEQ ID NO:3. In certain embodiments, the nucleic acid sequence is SEQ ID NO:4.

In another aspect, the invention provides a nucleic acid that has been reverse transcribed or amplified with an oligonucleotide of the invention. In certain embodiments, the nucleic acid is inserted into a vector. In certain embodiments, the vector is a resistance test vector.

### 4.6. Viruses and Viral Samples

Any virus known by one of skill in the art without limitation can be used as a source of patient-derived segments or viral sequences for use in the methods of the disclosure. In one embodiment of the disclosure, the virus includes viruses known to infect mammals, including dogs, cats, horses, sheep, cows, *etc.* In certain embodiments, the virus is known to infect primates. In preferred embodiments, the virus is known to infect humans. Examples of such viruses that infect humans include, but are not limited to, human immunodeficiency virus ("HIV"), herpes simplex virus, cytomegalovirus virus, varicella zoster virus, other human herpes viruses, influenza A, B and C virus, respiratory syncytial virus, hepatitis A, B and C viruses, hepatits B virus, hepatits C virus, rhinovirus, and human papilloma virus. In embodiments of the invention, the virus is HIV. Even more preferably, the virus is human immunodeficiency virus type 1 ("HIV-1"). In certain embodiments, the virus is human immunodeficiency virus type 2 ("HIV-2") The foregoing are representative of certain viruses for which there is presently available anti-viral chemotherapy and represent the viral families retroviridae, herpesviridae, orthomyxoviridae, paramxyxoviridae, picornaviridae, flaviviridae, pneumoviridae and hepadnaviridae. This disclosure can be used with other viral infections due to other viruses within these families as well as viral infections arising from viruses in other viral families for which there is or there is not a currently available therapy.

Viruses from which patient-derived segments or viral gene sequences are obtained can be found in a viral sample obtained by any means known in the art for obtaining viral samples. Such methods include, but are not limited to, obtaining a viral sample from a human or an animal infected with the virus or obtaining a viral sample from a viral culture. In one embodiment, the viral sample is obtained from a human individual infected with the virus. The viral sample could be obtained from any part of the infected individual's body or any secretion expected to contain the virus. Examples of such parts include, but are not limited to blood, serum, plasma, sputum, lymphatic fluid, semen, vaginal mucus and samples of other bodily fluids. In a preferred embodiment, the sample is a blood, serum or plasma sample.

In another embodiment, a patient-derived segment or viral gene sequence can be obtained from a virus that can be obtained from a culture. In some embodiments, the culture can be obtained from a laboratory. In other embodiments, the culture can be obtained from a collection, for example, the American Type Culture Collection.

In another embodiment, a patient-derived segment or viral gene sequence can be obtained from a genetically modified virus. The virus can be genetically modified using any method known in the art for genetically modifying a virus. For example, the virus can be grown for a desired number of generations in a laboratory culture. In one embodiment, no selective pressure is applied (*i.e.,* the virus is not subjected to a treatment that favors the replication of viruses with certain characteristics), and new mutations accumulate through random genetic drift. In another embodiment, a selective pressure is applied to the virus as it is grown in culture (*i.e.,* the virus is grown under conditions that favor the replication of viruses having one or more characteristics). In one embodiment, the selective pressure is an anti-viral treatment. Any known anti-viral treatment can be used as the selective pressure.

In certain embodiments, the virus is HIV and the selective pressure is a NNRTI. In another embodiment, the virus is HIV-1 and the selective pressure is a NNRTI. Any NNRTI can be used to apply the selective pressure. Examples of NNRTIs include, but are not limited to, nevirapine, delavirdine and efavirenz. By treating HIV cultured *in vitro* with a NNRTI, one can select for mutant HIV that have an increased resistance to the NNRTI. The stringency of the selective pressure can be manipulated to increase or decrease the survival of viruses not having the selected-for characteristic.

In other embodiments, the virus is HIV and the selective pressure is a NRTI. In another embodiment, the virus is HIV-1 and the selective pressure is a NRTI. Any NRTI can be used to apply the selective pressure. Examples of NRTIs include, but are not limited to, AZT, ddI, ddC, d4T, 3TC, and abacavir. By treating HIV cultured *in vitro* with a NRTI, one can select for mutant HIV that have an increased resistance to the NRTI. The stringency of the selective pressure can be manipulated to increase or decrease the survival of viruses not having the selected-for characteristic.

In still other embodiments, the virus is HIV and the selective pressure is a PI. In another embodiment, the virus is HIV-1 and the selective pressure is a PI. Any PI can be used to apply the selective pressure. Examples of PIs include, but are not limited to, saquinavir, ritonavir, indinavir, nelfinavir, amprenavir, lopinavir and atazanavir. By treating HIV cultured *in vitro* with a PI, one can select for mutant HIV that have an increased resistance to the PI. The stringency of the selective pressure can be manipulated to increase or decrease the survival of viruses not having the selected-for characteristic.

In still other embodiments, the virus is HIV and the selective pressure is an entry inhibitor. In another embodiment, the virus is HIV-1 and the selective pressure is an entry inhibitor. Any entry inhibitor can be used to apply the selective pressure. An example of a entry inhibitor includes, but is not limited to, fusion inhibitors such as, for example, enfuvirtide. Other entry inhibitors include co-receptor inhibitors, such as, for example, AMD3100 (Anormed). Such co-receptor inhibitors can include any compound that interferes with an interaction between HIV and a co-receptor, e.g., CCR5 or CRCX4, without limitation. By treating HIV cultured *in vitro* with an entry inhibitor, one can select for mutant HIV that have an increased resistance to the entry inhibitor. The stringency of the selective pressure can be manipulated to increase or decrease the survival of viruses not having the selected-for characteristic.

In yet other embodiments, the virus is HIV and the selective pressure is an integrase inhibitor. In certain embodiment, the virus is HIV-1 and the selective pressure is an integrase inhibitor. Any integrase inhibitor can be used to apply the selective pressure. Examples of integrase inhibitors include, but are not limited to, L-731,988 and L-870,810. By treating HIV cultured *in vitro* with an integrase inhibitor, one can select for mutant HIV that have an increased resistance to the inhibitor. The stringency of the selective pressure can be manipulated to increase or decrease the survival of viruses not having the selected-for characteristic.

In another aspect, the patient-derived segment or viral gene sequence can be made by mutagenizing a virus, a viral genome, or a part of a viral genome. Any method of mutagenesis known in the art can be used for this purpose. In certain embodiments, the mutagenesis is essentially random. In certain embodiments, the essentially random mutagenesis is performed by exposing the virus, viral genome or part of the viral genome to a mutagenic treatment. In another embodiment, a gene that encodes a viral protein that is the target of an anti-viral therapy is mutagenized. Examples of essentially random mutagenic treatments include, for example, exposure to mutagenic substances (*e.g*., ethidium bromide, ethylmethanesulphonate, ethyl nitroso urea (ENU) *etc*.) radiation (*e.g.,* ultraviolet light), the insertion and/or removal of transposable elements (*e.g*., Tn5, Tn10), or replication in a cell, cell extract, or *in vitro* replication system that has an increased rate of mutagenesis. *See, e.g.,* Russell et al., 1979, Proc. Nat. Acad. Sci. USA 76:5918-5922; Russell, W., 1982, Environmental Mutagens and Carcinogens: Proceedings of the Third International Conference on Environmental Mutagens. One of skill in the art will appreciate that while each of these methods of mutagenesis is essentially random, at a molecular level, each has its own preferred targets.

In another aspect, the patient-derived segment or viral gene sequence can be made using site-directed mutagenesis. Any method of site-directed mutagenesis known in the art can be used (*see e.g.,* Sambrook et al., 2001, Molecular Cloning: A Laboratory Manual, Cold Spring Harbor Laboratory, 3rd ed., NY; and Ausubel et al., 2005, Current Protocols in Molecular Biology, Greene Publishing Associates and Wiley Interscience, NY, and Sarkar and Sommer, 1990, Biotechniques, 8:404-407). The site directed mutagenesis can be directed to, *e.g*., a particular gene or genomic region, a particular part of a gene or genomic region, or one or a few particular nucleotides within a gene or genomic region. In one embodiment, the site directed mutagenesis is directed to a viral genomic region, gene, gene fragment, or nucleotide based on one or more criteria. In one embodiment, a gene or a portion of a gene is subjected to site-directed mutagenesis because it encodes a protein that is known or suspected to be a target of an anti-viral therapy, *e.g*., the gene encoding HIV reverse transcriptase, intergrase, or a portion thereof such as RNAse H. In another embodiment, a portion of a gene, or one or a few nucleotides within a gene, are selected for site-directed mutagenesis. In one embodiment, the nucleotides to be mutagenized encode amino acid residues that are known or suspected to interact with an anti-viral compound. In another embodiment, the nucleotides to be mutagenized encode amino acid residues that are known or suspected to be mutated in viral strains that are resistant or susceptible or hypersusceptible to one or more antiviral agents. In another embodiment, the mutagenized nucleotides encode amino acid residues that are adjacent to or near in the primary sequence of the protein residues known or suspected to interact with an anti-viral compound or known or suspected to be mutated in viral strains that are resistant or susceptible or hypersusceptible to one or more antiviral agents. In another embodiment, the mutagenized nucleotides encode amino acid residues that are adjacent to or near to in the secondary, tertiary or quaternary structure of the protein residues known or suspected to interact with an anti-viral compound or known or suspected to be mutated in viral strains having an altered replication capacity. In another embodiment, the mutagenized nucleotides encode amino acid residues in or near the active site of a protein that is known or suspected to bind to an anti-viral compound.

### 5. EXAMPLES

### 5.1. Example 1: Measuring Anti-HIV Drug Susceptibility

This example provides methods and compositions for accurately and reproducibly measuring the resistance or sensitivity of HIV infecting a patient to antiretroviral drugs including, for example, IN inhibitors such as L-870,810. The methods for measuring resistance or susceptibility to such drugs can be adapted to other viruses, including, but not limited to hepadnaviruses *(e.g.,* human hepatitis B virus), flaviviruses *(e.g.,* human hepatitis C virus) and herpesviruses *(e.g*., human cytomegalovirus). The methods described in this example can also be used to determine the replication capacity of the HIV.

The drug resistance tests described herein are a modification of the methods for phenotypic drug susceptibility and resistance tests described in US Patent Number 5,837,464 (International Publication Number WO 97/27319).

### 5.1.1. Construction of Test Vector Libraries

Patient-derived segment(s) corresponding to either the entire *pol* gene, encoding HIV protease, reverse transcriptase, integrase, and RNAse H (hereinafter "POL"), or the portion of *pol* encoding amino acids 319-440 of reverse transcriptase, integrase, and RNAse H (hereinafter "RHIN"), were amplified by the reverse transcription-polymerase chain reaction method (RT-PCR) using viral RNA isolated from viral particles present in the plasma or serum of HIV-infected individuals as follows. Virus was pelleted by centrifugation at 20,400 x g for 60 min from plasma (typically, 1 ml) prepared from blood samples collected in evacuated tubes containing either EDTA, acid-citrate dextrose, or heparin as an anticoagulant. Virus particles were disrupted by resuspending the pellets in 200 Ill of lysis buffer (4 M guanidine thiocyanate, 0.1 M Tris HC1 [pH 8.0], 0.5% sodium lauryl sarcosine, 1% dithiothreitol). RNA was extracted from viral lysates by using oligo(dT) linked to magnetic beads (Dynal, Oslo, Norway). Reverse transcription was performed with Superscript III (Invitrogen) at 50 degrees for 1 hour using primer 1. All primer sequences are listed in Table 1, below.

| **TABLE 1** | | | | | |
|---|---|---|---|---|---|
| **Reverse Transcriptase Primer** | | | | | |
| Name | SEQ ID NO. | Gene Primer is Located in | Sequence | Amplicon | |
| Primer 1 | SEQ ID NO:1 | *vif* | 5' CTTTCCTCGAGAYATACATATGGTGT 3' | POL and RHIN | |
| | | | | | |

| | **PCR Primers** | | | | |
|---|---|---|---|---|---|
| Name | | Gene Primer is Located in | Sequence | Amplicon | Direction |
| Primer 2 | SEQ ID NO:2 | *pol* | 5' CAGRGARATTCTAAAAGAACCGGTACATGG 3' | RHIN | 5' |
| Primer 3 | SEQ ID NO:3 | *gag* | 5' TTGCAGGGCCCCTAGRAAAAARGGCTG 3' | POL | 5' |
| Primer 4 | SEQ ID NO:4 | *vif* | 5' CTTTCCTCGAGAYATACATATGGTGTTTTAC 3' | POL and RHIN | 3' |

From the resultant cDNA either POL or RHIN sequences were amplified using the Advantage High Fidelity PCR kit (BD Biosciences; Clontech). POL amplification products are made using forward Primer 3 containing an ApaI site and reverse Primer 4 containing a Xho 1 site. RHIN amplification products are made using forward Primer 2 containing a PINA1 site and reverse Primer 4 containing a Xho 1 site. PCR cycling involves 40 cycles of a 3 step program according to the protocol shown in Table 2, below.

**TABLE 2**

| **AMPLIFICATION PROTOCOL FOR RHIN** | | |
|---|---|---|
| **PCR PROFILE** | **DEGREES** | **MINUTES** |
| DENATURE | 94 | 2:00 |
| **40 CYCLES OF:** | | |
| DENATURE | 94 | 0:40 |
| ANNEAL | 60 | 1:00 |
| EXTEND | 72 | 2:00 |
| | | |
| EXTENTSION | 72 | 10:00 |
| HOLD | 4 | INDEF |
| | | |

| **AMPLIFICATION OF POL** | | |
|---|---|---|
| **PCR PROFILE** | **DEGREES** | **MINUTES** |
| DENATURE | 94 | 2:00 |
| **40 CYCLES OF:** | | |
| DENATURE | 94 | 0:40 |
| ANNEAL | 58 | 1:00 |
| EXTEND | 72 | 3:00 |
| | | |
| EXTENTSION | 72 | 10:00 |
| HOLD | 4 | INDEF |

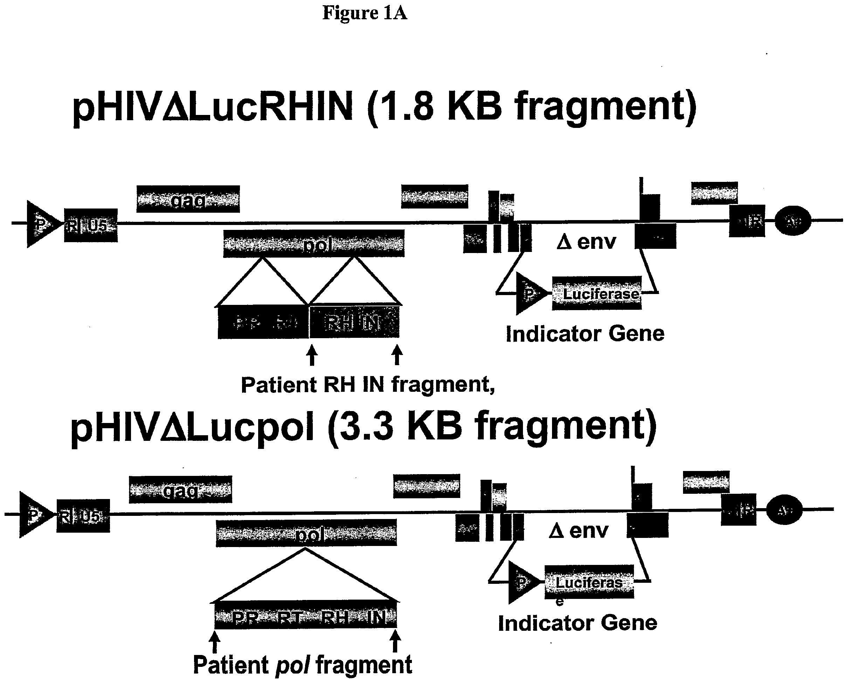

A retroviral vector designed to measure antiretroviral drug susceptibility was constructed by using an infectious molecular clone of HIV-1. The vector, referred to herein as an indicator gene viral vector (IGW), is replication defective and contains a luciferase expression cassette inserted within a deleted region of the envelope *(env)* gene. The IGVV is described in US Patent Number 5,837,464 (International Publication Number WO 97/27319). This retroviral vector was further modified to allow insertion of either the entire *pol* gene (POL) or the portion of *pol* encoding amino acids 319-440 of reverse transcriptase, the RNase H portion of reverse transcriptase, and integrase (RHIN) by engineering an *Xho*1 restriction enzyme recognition site into *vif.* Prior to doing this, an *Xho* 1 site in *nef* was deleted. Test vectors (TVs) were constructed by incorporating amplified POL or RHIN into the IGVV by using *Apa*I and *Xho*1 or *Pin*AI and *Xho*1 restriction sites respectively. TVs were prepared as libraries (pools) in order to capture and preserve the *pol* or RHIN sequence heterogeneity of the virus in the patient. POL amplification products were digested with *Apa*I and *Xho1,* purified by agarose gel electrophoresis, and ligated to *Apa*I- and *Xho1-*digested IGVV DNA. RHIN amplification products were digested with *PinA*I and *Xho1,* purified by agarose gel electrophoresis, and ligated to *PinA*I and *Xho1*-digested IGVV DNA. Diagrammatic representations of these constructs are presented as Figure 1a. Ligation reactions were used to transform competent *Escherichia coli* (Invitrogen, Carlsbad, Calif.). An aliquot of each transformation was plated onto agar, and colony counts were used to estimate the number of patient-derived segments represented in each TV library. TV libraries that comprised less than 50 members are not considered representative of the patient virus.

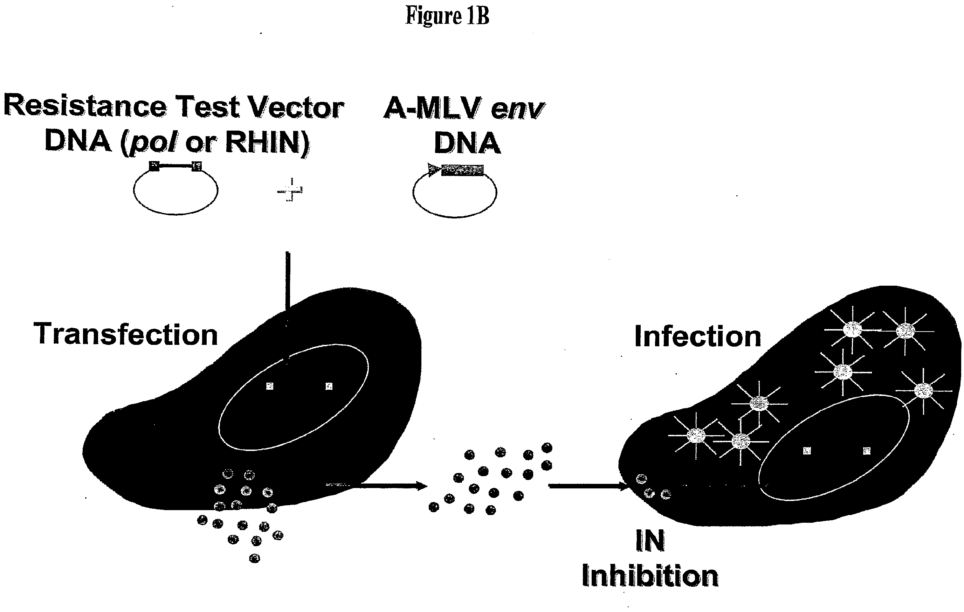

A packaging expression vector encoding an amphotrophic MuLV 4070A *env* gene product (described in US Patent Number 5,837,464) enables production in a host cell of viral particles which can efficiently infect human target cells *(see* Figure 1b). TV libraries encoding all HIV genes with the exception of *env,* produced as described above, were used to transfect a packaging host cell. The packaging expression vector which encodes the amphotrophic MuLV 4070A *env* gene product is used with the resistance test vector to enable production of infectious pseudotyped viral particles comprising the resistance test vector libraries.

### 6.1.2. Anti-HIV Drug Susceptibility Assays

Drug susceptibility tests performed with test vectors were carried out using packaging host and target host cells consisting of the human embryonic kidney cell line 293.

Susceptibility tests were carried out with the TV libraries by using viral particles comprising the RTV libraries to infect a host cell in which the expression of the indicator gene is measured. The amount of indicator gene (luciferase) activity detected in infected cells is used as a direct measure of "infectivity," *i.e.,* the ability of the virus to complete a single round of replication. Thus, drug resistance or sensitivity can be determined by plotting the amount of luciferase activity produced by patient derived viruses in the presence of varying concentrations of the antiviral drug. By identifying the concentration of drug at which luciferase activity is half-maximum, the IC₅₀ of the virus from which patient-derived segment(s) were obtained for the antiretroviral agent can be determined. The IC₅₀ provides a direct measure of the resistance or susceptibility of the HIV infecting the patient to the anti-viral drug.

In the susceptibility tests, packaging host (293) cells were seeded in 10-cm-diameter dishes and were transfected one day after plating with test vector plasmid DNA and the envelope expression vector. Transfections were performed using a calcium-phosphate co-precipitation procedure. The cell culture media containing the DNA precipitate was replaced with fresh medium, from one to 24 hours, after transfection. Cell culture medium containing viral particles comprising the TV libraries was harvested one to four days after transfection and was passed through a 0.45-mm filter before optional storage at -80 °C. Before infection, host cells (293 cells) to be infected were plated in cell culture media containing varying concentrations of L-870,810, the anti-HIV agent to be tested in the assay. Control infections were performed using cell culture media from mock transfections (no DNA) or transfections containing the test vector plasmid DNA without the envelope expression plasmid. One to three or more days after infection the media was removed and cell lysis buffer (Promega Corp.; Madison, WI) was added to each well. Cell lysates were assayed for luciferase activity. Alternatively, cells were lysed and luciferase was measured by adding Steady-Glo (Promega Corp.; Madison, WI) reagent directly to each well without aspirating the culture media from the well. The amount of luciferase activity produced in infected cells was normalized to adjust for variation in transfection efficiency in the transfected host cells by measuring the luciferase activity in the transfected cells, which is not dependent on viral gene functions, and adjusting the luciferase activity from infected cell accordingly. The normalized luciferase activity was then plotted as a function of the log of anti-HIV agent present to determine the IC₅₀ of the assayed HIV.

### 6.1.3. HIV Replication Capacity Assays

Replication capacity tests performed with test vectors were carried out using packaging host and target host cells consisting of the human embryonic kidney cell line 293.

Replication capacity tests were carried out with the TV libraries by using viral particles comprising the RTV libraries to infect a host cell in which the expression of the indicator gene is measured. The amount of indicator gene (luciferase) activity detected in infected cells is used as a direct measure of "infectivity," *i.e.,* the ability of the virus to complete a single round of replication. Thus, the amount of luciferase activity observed in the infected cells provides a direct measurement of the replication capacity of the virus. By determining the amount of luciferase activity, the replication capacity of the virus from which patient-derived segment(s) were obtained for the antiretroviral agent can be determined. The amount of luciferase activity observed can also be compared to the amount of luciferase activity observed for a control assay performed with a reference viral segment, such as an viral segment obtained from a reference virus such as, for example, NL4-3 or IIIB. When such comparisons are performed, the replication capacity of the virus or viral population can be reported as a percentage of the replication capacity observed for the reference virus.

In the replication capacity tests, packaging host (293) cells were seeded in 10-cm-diameter dishes and were transfected one day after plating with test vector plasmid DNA and the envelope expression vector. Transfections were performed using a calcium-phosphate co-precipitation procedure. The cell culture media containing the DNA precipitate was replaced with fresh medium, from one to 24 hours, after transfection. Cell culture medium containing viral particles comprising the TV libraries was harvested one to four days after transfection and was passed through a 0.45-mm filter before optional storage at -80 °C. Before infection, host cells (293 cells) to be infected were plated in cell culture media. Control infections were performed using cell culture media from mock transfections (no DNA) or transfections containing the test vector plasmid DNA without the envelope expression plasmid. One to three or more days after infection the media was removed and cell lysis buffer (Promega Corp.; Madison, WI) was added to each well. Cell lysates were assayed for luciferase activity. Alternatively, cells were lysed and luciferase was measured by adding Steady-Glo (Promega Corp.; Madison, WI) reagent directly to each well without aspirating the culture media from the well. The amount of luciferase activity produced in infected cells was normalized to adjust for variation in transfection efficiency in the transfected host cells by measuring the luciferase activity in the transfected cells, which is not dependent on viral gene functions, and adjusting the luciferase activity from infected cell accordingly.

### 6.1.4. Results of Anti-HIV Drug Susceptibility Assays

The assays described in the Examples above were used to assess the susceptibility of several HIV mutants comprising site-directed mutations and HIV isolated from 45 treatment-naive HIV-infected patients. The results of these assays are described below.

First, the susceptibility assays were used to assess the susceptibility of several HIV mutants comprising site-directed mutations in the portion of *pol* encoding integrase. The integrase mutations tested were F121Y, T125K, V151I, S153Y, M154I, N155S, F121Y/T125K, T66I/M154I, T66I/S153Y, V72I/T125K/F121Y, and V72I/T125K/F121Y/V151I. These mutations were selected based on the report that such mutations are associated with resistance to L-870,810 and the diketo acid L-731,988. *See* Hazuda et al., 2004, P.N.A.S. USA 101:11233-11238.

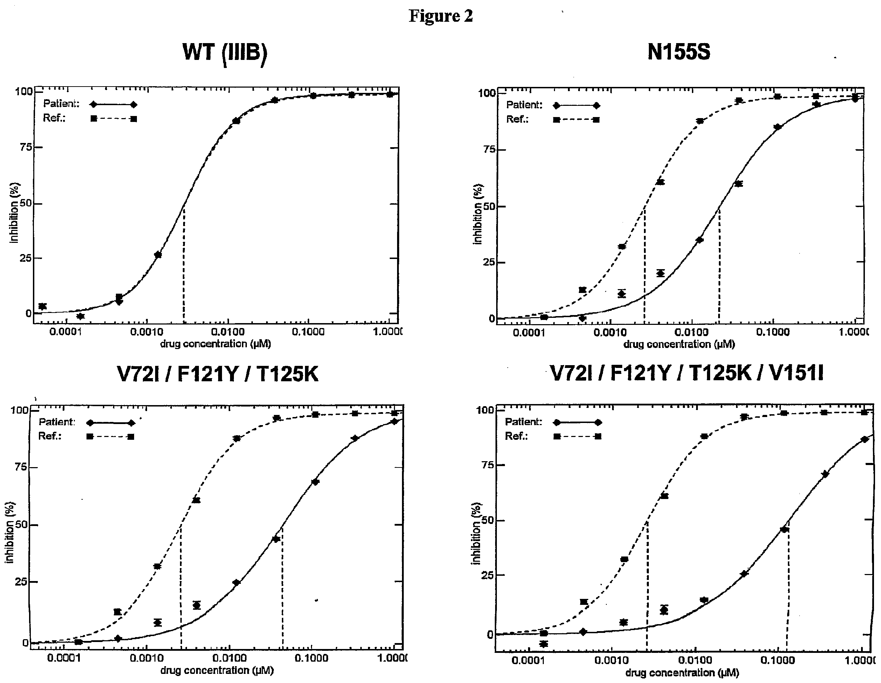

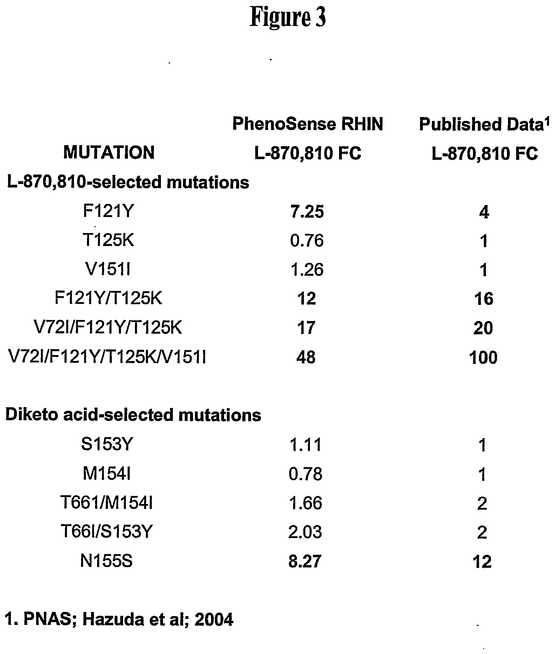

The results of the susceptibility tests for the site directed mutants are presented in Figures 2 and 3. Figure 2 demonstrates that site-directed mutants N155S, V72I/F121Y/T125K, and V72I/T125K/F121Y/V151I each demonstrate reduced susceptibility to L-870,810 in the susceptibility tests described above. Reduced susceptibility is shown by the increased IC₅₀ observed for the site-directed mutants. This result is consistent with the results reported by Hazuda *et al., supra.* Similarly, the fold change (FC) observed for the IC₅₀s of the site-directed mutants in the assays described above is consistent with the results reported by Hazuda *et al.,* as shown in Figure 3. Taken together, these results indicate that the drug susceptibility assays described above can accurately determine the resistance or susceptibility of an HIV to IN inhibitors.

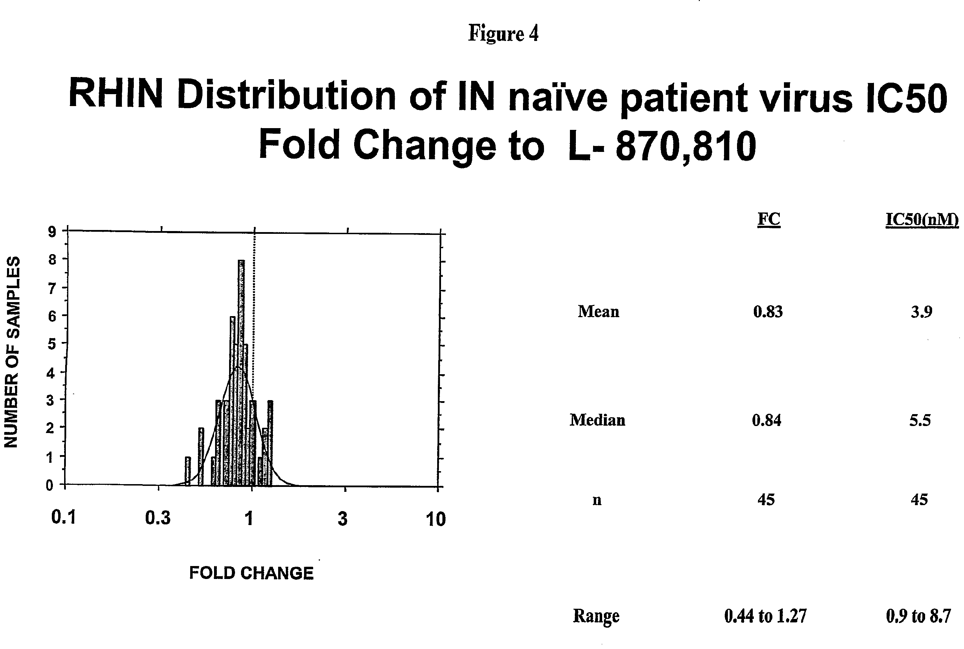

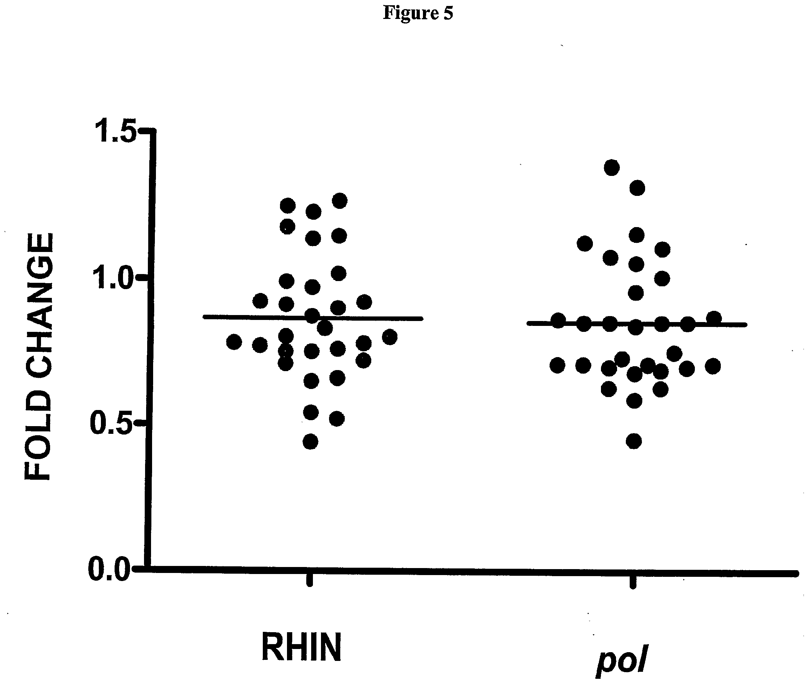

The results of the assays testing the susceptibility of HIV isolated from 45 treatment-naïve HIV-infected patients are presented in Figures 4 and 5. In the assays, RHIN sequences from 45 patient viruses were successfully amplified and tested for susceptibility to L-870,810. The distribution of IC₅₀ FC to L-870,810 for all tested viruses was narrow, with mean FC 0.83 and observed FC ranging from 0.44 to 1.27. *See* Figure 4. No significant differences in L-870,810 susceptibility and no known IN resistance mutations were observed when comparing vectors containing either RHIN or POL fragments from the primary patient virus, as shown by Figure 5. Thus, both RHIN and POL yielded concordant results for particular patient isolates.

### 6.1.5. Replication Capacity Assay Results

In addition to drug susceptibility assays, replication capacity assays were performed to assess the replication capacity of the 45 viral isolates from treatment-naive patients as well as the N155S site directed mutant. In the assays, replication capacity (RC) in the absence of drug was measured and variation in RHIN for patient viruses was detected. The replication capacity measured using the RHIN segment was also compared to the replication capacity observed when assayed using the PR-RT segment described in U.S. Patent 5,837,464 as described therein to assess the concordance of the two assays.

The replication capacity assays using the RHIN segment, and the comparison to replication capacities observed with the PR-RT segment is shown in Figure 6. The median replication capacity observed for the RHIN segment was 63%, and the observed replication capacities ranged from 11%-152%. In addition, the impaired replication of the N155S mutant (50%) reported by Hazuda *et al.* was confirmed, as this virus exhibited a replication capacity of 30% (data not shown). No significant differences in RC was observed when comparing vectors containing either RHIN or PR-RT fragments from the same virus, as shown in Figure 6.

The examples provided herein, both actual and prophetic, are merely embodiments of the present invention and are not intended to limit the invention in any way.

## Claims

1. A method for determining the susceptibility of a patient's human immunodeficiency virus (HIV) to an integrase inhibitor, the method comprising:
a) obtaining a patient-derived segment from the patient's HIV, wherein the patient-derived segment comprises a nucleic acid that encodes HIV reverse transcriptase, RNase H and integrase;
b) culturing a host cell in the presence of the integrase inhibitor, wherein the host cell comprises the patient-derived segment from the patient's HIV and an indicator gene, wherein the activity of the indicator gene depends on the activity of the HIV integrase;
c) measuring the activity of the indicator gene in the host cell; and
d) comparing the activity of the indicator gene as measured in step c) with a reference activity of the indicator gene,
wherein the difference between the activity of the indicator gene measured in step c) relative to the reference activity correlates with the susceptibility of the HIV to the integrase inhibitor, thereby determining the susceptibility of the HIV to the integrase inhibitor.

2. The method of Claim 1, wherein the reference activity of the indicator gene is determined by performing the method of Claim 1 in the absence of the integrase inhibitor.

3. The method of Claim 1 or 2, wherein the reference activity of the indicator gene is determined by performing the method of Claim 1 with a reference viral segment.

4. The method of Claim 1, wherein the HIV is determined to have reduced susceptibility to the integrase inhibitor.

5. The method of Claim 1, wherein the HIV is determined to have increased susceptibility to the integrase inhibitor.

6. The method of Claim 1, wherein the patient-derived segment is about 3.3 kb in length.

7. The method of Claim 1, wherein the patient-derived segment has been prepared in a reverse transcription or polymerase chain reaction (PCR) reaction.

8. The method of Claim 7, wherein the reverse transcription or PCR reaction comprises an oligonucleotide comprising a nucleic acid sequence that is SEQ ID NO:1, SEQ ID NO:2, SEQ ID NO:3, or SEQ ID NO:4.

9. The method of Claim 1, wherein the method additionally comprises the step of infecting the host cell with a viral particle comprising the patient-derived segment and the indicator gene, wherein the step of infecting is performed prior to step c) of the method of Claim 1.

10. The method of Claim 1, wherein the indicator gene is a luciferase gene.

11. The method of Claim 1, wherein the indicator gene is a lacZ gene.

12. The method of Claim 1, wherein the host cell is a human cell.

13. The method of Claim 1, wherein the host cell is a human embryonic kidney cell.

14. The method of Claim 1, wherein the host cell is a 293 cell.

15. The method of Claim 1, wherein the host cell is a human T cell.

16. The method of Claim 1, wherein the host cell is derived from a human T cell leukemia cell line.

17. The method of Claim 1, wherein the host cell is a Jurkat cell.

18. The method of Claim 1, wherein the host cell is a H9 cell.

19. The method of Claim 1, wherein the host cell is a CEM cell.

20. A method for determining resistance of an HIV infecting a patient to an integrase inhibitor, comprising:
a) determining the susceptibility of the HIV to the integrase inhibitor according to the method of Claim 1; and
b) comparing the susceptibility of the HIV to the integrase inhibitor determined in step a) with a standard curve of susceptibility of the HIV to the integrase inhibitor,
wherein a decrease in the susceptibility of the HIV to the integrase inhibitor relative to the standard curve indicates that the HIV is resistant to the integrase inhibitor and the amount of the decrease in susceptibility of the HIV to the integrase inhibitor indicates the degree to which the HIV is resistant to the integrase inhibitor.

21. A method for determining the progression or development of resistance of an HIV infecting a patient to an integrase inhibitor, comprising:
a) determining the susceptibility of the HIV to the integrase inhibitor at a first time according to the method of Claim 1, wherein the patient-derived segment is obtained from the patient at about the first time;
b) assessing the effectiveness of the integrase inhibitor as performed in step a) at a later second time; and
c) comparing the effectiveness of the integrase inhibitor assessed in steps a) and b),
wherein a decrease in the susceptibility of the HIV to the integrase inhibitor at the later second time as compared to the first time indicates development or progression of integrase inhibitor resistance in the HIV infecting the patient.

## Patentansprüche

1. Verfahren zur Bestimmung der Suszeptibilität des Humanimmundefizienzvirus (HIV) eines Patienten für einen Integraseinhibitor, wobei das Verfahren Folgendes umfasst:
a) Erhalten eines vom Patienten stammenden Segments aus dem HIV des Patienten, worin das vom Patienten stammende Segment eine Nukleinsäure umfasst, die für HIV-Reverstranskriptase, RNase H und Integrase codiert;
b) Kultivieren einer Wirtszelle in der Gegenwart des Integraseinhibitors, worin die Wirtszelle das vom Patienten stammende Segment aus dem HIV des Patienten und ein Indikatorgen umfasst, worin die Aktivität des Indikatorgens von der Aktivität der HIV-Integrase abhängt;
c) Messen der Aktivität des Indikatorgens in der Wirtszelle; und
d) Vergleichen der Aktivität des Indikatorgens, wie in Schritt c) gemessen, mit einer Referenzaktivität des Indikatorgens,
worin die Differenz zwischen der in Schritt c) gemessenen Aktivität des Indikatorgens relativ zur Referenzaktivität mit der Suszeptibilität des HIV für den Integraseinhibitor korreliert, wodurch die Suszeptibilität des HIV für den Integraseinhibitor bestimmt wird.

2. Verfahren nach Anspruch 1, worin die Referenzaktivität des Indikatorgens durch Durchführung des Verfahrens nach Anspruch 1 in der Abwesenheit des Integraseinhibitors bestimmt wird.

3. Verfahren nach Anspruch 1 oder 2, worin die Referenzaktivität des Indikatorgens durch Durchführung des Verfahrens nach Anspruch 1 mit einem viralen Referenzsegment bestimmt wird.

4. Verfahren nach Anspruch 1, worin bestimmt wird, dass das HIV reduzierte Suszeptibilität für den Integraseinhibitor aufweist.

5. Verfahren nach Anspruch 1, worin bestimmt wird, dass das HIV erhöhte Suszeptibilität für den Integraseinhibitor aufweist.

6. Verfahren nach Anspruch 1, worin das vom Patienten stammende Segment etwa 3,3 kb lang ist.

7. Verfahren nach Anspruch 1, worin das vom Patienten stammende Segment in einer Reverstranskriptionsreaktion oder Polymerasekettenreaktion (PCR) hergestellt wurde.

8. Verfahren nach Anspruch 7, worin die Reverstranskriptionsreaktion oder PCR ein Oligonukleotid umfassend eine Nukleinsäuresequenz, die SEQ ID Nr. 1, SEQ ID Nr. 2, SEQ ID Nr. 3 oder SEQ ID Nr. 4 ist, umfasst.

9. Verfahren nach Anspruch 1, worin das Verfahren zusätzlich den Schritt des Infizierens der Wirtszelle mit einem viralen Partikel umfassend das vom Patienten stammende Segment und das Indikatorgen umfasst, worin der Schritt des Infizierens vor Schritt c) des Verfahrens nach Anspruch 1 durchgeführt wird.

10. Verfahren nach Anspruch 1, worin das Indikatorgen ein Luciferase-Gen ist.

11. Verfahren nach Anspruch 1, worin das Indikatorgen ein lacZ-Gen ist.

12. Verfahren nach Anspruch 1, worin die Wirtszelle eine Humanzelle ist.

13. Verfahren nach Anspruch 1, worin die Wirtszelle eine humanembryonische Nierenzelle ist.

14. Verfahren nach Anspruch 1, worin die Wirtszelle eine 293-Zelle ist.

15. Verfahren nach Anspruch 1, worin die Wirtszelle eine humane T-Zelle ist.

16. Verfahren nach Anspruch 1, worin die Wirtszelle aus einer humanen T-Zell-Leukämie-Zelllinie stammt.

17. Verfahren nach Anspruch 1, worin die Wirtszelle eine Jurkat-Zelle ist.

18. Verfahren nach Anspruch 1, worin die Wirtszelle eine H9-Zelle ist.

19. Verfahren nach Anspruch 1, worin die Wirtszelle eine CEM-Zelle ist.

20. Verfahren zur Bestimmung der Resistenz eines einen Patienten infizierenden HIV für einen Integraseinhibitor, umfassend:
a) Bestimmen der Suszeptibilität des HIV für den Integraseinhibitor nach dem Verfahren nach Anspruch 1; und
b) Vergleichen der Suszeptibilität des HIV für den in Schritt a) bestimmten Integraseinhibitor mit einer Standardkurve der Suszeptibilität des HIV für den Integraseinhibitor,
worin eine Verringerung der Suszeptibilität des HIV für den Integraseinhibitor relativ zur Standardkurve angibt, dass das HIV gegen den Integraseinhibitor resistent ist, und der Betrag der Abnahme der Suszeptibilität des HIV für den Integraseinhibitor den Grad der Resistenz des HIV gegen den Integraseinhibitor angibt.

21. Verfahren zur Bestimmung der Progression oder Entwicklung von Resistenz eines einen Patienten infizierenden HIV gegen einen Integraseinhibitor, umfassend:
a) Bestimmen der Suszeptibilität des HIV für den Integraseinhibitor zu einem ersten Zeitpunkt nach dem Verfahren nach Anspruch 1, worin das vom Patienten stammende Segment aus dem Patienten etwa zum ersten Zeitpunkt erhalten wird;
b) Bewerten der Wirksamkeit des Integraseinhibitors, wie in Schritt a) durchgeführt, zu einem späteren zweiten Zeitpunkt; und
c) Vergleichen der in Schritt a) und b) bewerteten Wirksamkeit des Integraseinhibitors,
worin eine Abnahme der Suszeptibilität des HIV für den Integraseinhibitor zum späteren zweiten Zeitpunkt im Vergleich zum ersten Zeitpunkt die Entwicklung oder Progression von Integraseinhibitorresistenz im den Patienten infizierenden HIV angibt.

## Revendications

1. Un procédé permettant de déterminer la sensibilité du virus de l'immunodéficience humaine (VIH) d'un patient à un inhibiteur de l'intégrase, le procédé consistant à :
a) obtenir un segment dérivé d'un patient provenant du VIH du patient, dans lequel le segment dérivé d'un patient comprend un acide nucléique qui code la transcriptase inverse, l'ARNase h et l'intégrase du VIH ;
b) cultiver une cellule hôte en présence de l'inhibiteur de l'intégrase, dans lequel la cellule hôte comprend le segment dérivé d'un patient provenant du VIH du patient et un gène indicateur, dans lequel l'activité du gène indicateur dépend de l'activité de l'intégrase du VIH ;
c) mesurer l'activité du gène indicateur dans la cellule hôte ; et
d) comparer l'activité du gène indicateur telle que mesurée à l'étape c) avec une activité de référence du gène indicateur,
dans lequel la différence entre l'activité du gène indicateur mesurée à l'étape c) par rapport à l'activité de référence est en corrélation avec la sensibilité du VIH à l'inhibiteur de l'intégrase, permettant ainsi de déterminer la sensibilité du VIH à l'inhibiteur de l'intégrase.

2. Le procédé selon la revendication 1, dans lequel l'activité de référence du gène indicateur est déterminée en exécutant le procédé selon la revendication 1 en l'absence de l'inhibiteur de l'intégrase.

3. Le procédé selon la revendication 1 ou 2, dans lequel l'activité de référence du gène indicateur est déterminée en exécutant le procédé selon la revendication 1 avec une référence virale du segment.

4. Le procédé selon la revendication 1, dans lequel le VIH est déterminé pour avoir une sensibilité réduite à l'inhibiteur de l'intégrase.

5. Le procédé selon la revendication 1, dans lequel le VIH est déterminé pour avoir une sensibilité augmentée à l'inhibiteur de l'intégrase.

6. Le procédé selon la revendication 1, dans lequel le segment dérivé d'un patient a une longueur d'environ 3,3 kb.

7. Le procédé selon la revendication 1, dans lequel le segment dérivé d'un patient a été préparé dans une réaction de transcription inverse ou une réaction en chaîne par polymérase (PCR).

8. Le procédé selon la revendication 7, dans lequel la réaction de transcription inverse ou la réaction PCR comprend un oligonucléotide comprenant une séquence d'acides nucléiques qui est SEQ ID NO:1, SEQ ID NO:2, SEQ ID NO:3 ou SEQ ID NO:4.

9. Le procédé selon la revendication 1, dans lequel le procédé comprend en outre l'étape consistant à infecter la cellule hôte avec une particule virale comprenant le segment dérivé d'un patient et le gène indicateur, dans lequel l'étape d'infection est exécutée avant l'étape c) du procédé selon la revendication 1.

10. Le procédé selon la revendication 1, dans lequel le gène indicateur est un gène de la luciférase.

11. Le procédé selon la revendication 1, dans lequel le gène indicateur est un gène lacZ.

12. Le procédé selon la revendication 1, dans lequel la cellule hôte est une cellule humaine.

13. Le procédé selon la revendication 1, dans lequel la cellule hôte est une cellule rénale embryonnaire humaine.

14. Le procédé selon la revendication 1, dans lequel la cellule hôte est une cellule 293.

15. Le procédé selon la revendication 1, dans lequel la cellule hôte est un lymphocyte T humain.

16. Le procédé selon la revendication 1, dans lequel la cellule hôte est dérivée d'une lignée de cellules leucémiques de lymphocytes T humains.

17. Le procédé selon la revendication 1, dans lequel la cellule hôte est une cellule Jurkat.

18. Le procédé selon la revendication 1, dans lequel la cellule hôte est une cellule H9.

19. Le procédé selon la revendication 1, dans lequel la cellule hôte est une cellule CEM.

20. Un procédé permettant de déterminer la résistance d'un VIH infectant un patient à un inhibiteur de l'intégrase, consistant à :
a) déterminer la sensibilité du VIH à l'inhibiteur de l'intégrase selon le procédé de la revendication 1 ; et
b) comparer la sensibilité du VIH à l'inhibiteur de l'intégrase déterminée à l'étape a) avec une courbe standard de la sensibilité du VIH à l'inhibiteur de l'intégrase,
dans lequel une diminution de la sensibilité du VIH à l'inhibiteur de l'intégrase par rapport à la courbe standard indique que le VIH est résistant à l'inhibiteur de l'intégrase et l'ampleur de la diminution de la sensibilité du VIH à l'inhibiteur de l'intégrase indique le degré dans lequel le VIH est résistant à l'inhibiteur de l'intégrase.

21. Un procédé permettant de déterminer la progression ou le développement de la résistance d'un VIH infectant un patient à un inhibiteur de l'intégrase, consistant à :
a) déterminer la sensibilité du VIH à l'inhibiteur de l'intégrase lors d'une première fois selon le procédé de la revendication 1, dans lequel le segment dérivé d'un patient est obtenu du patient environ lors de la première fois ;
b) évaluer l'efficacité de l'inhibiteur de l'intégrase telle qu'exécutée à l'étape a) lors d'une deuxième fois ultérieure ; et
c) comparer l'efficacité de l'inhibiteur de l'intégrase évaluée aux étapes a) et b),
dans lequel une diminution de la sensibilité du VIH à l'inhibiteur de l'intégrase lors de la deuxième fois ultérieure par comparaison avec la première fois indique le développement ou la progression de la résistance à l'inhibiteur de l'intégrase dans le VIH infectant le patient.
